# EUROPEAN PATENT APPLICATION

(11) **EP 3 828 292 A1**
(43) Date of publication of application: **02.06.2021**
(21) Application number: 20206400.2
(22) Date of filing: 21.07.2016
(51) Int. Cl.: C12Q 1/6886, C12Q 1/6827, C40B 40/06, C40B 30/04, C12P 19/34

(54) **LOCKED NUCLEIC ACIDS FOR CAPTURING FUSION GENES**

(30) Priority: 21.07.2015 US 201562195280 P
(62) Divisional of application: 16828575.7
(71) Applicant: Guardant Health, Inc., Redwood City, CA 94063 (US)
(72) Inventor: MORTIMER, Stefanie Ann, Ward, Morgan Hill, CA 95037 (US)
(74) Representative: Marshall, Cameron John

(57) **Abstract**

Provided herein is a method for enriching a sample for polynucleotides comprising a breakpoint of a fusion gene, comprising: a) contacting a probe set comprising a plurality of polynucleotide probes, each probe configured to specifically hybridize to a fusion gene, wherein the set comprises one or more high affinity polynucleotide probes (e.g., a polynucleotide comprising one or more locked nucleic acid nucleotides), with a mixture of polynucleotides under hybridization conditions to produce probe-captured polynucleotides; and b) isolating the probe-captured polynucleotides from the mixture, to produce a sample enriched with polynucleotides comprising breakpoint fragments of the fusion gene.

## Description

### CROSS-REFERENCE

This application claims priority to U.S. Provisional Patent Application No. 62/195,280, filed July 21, 2015, which is entirely incorporated herein by reference.

### BACKGROUND

Gene fusion events are chromosomal rearrangements that bring together formerly separate portions of at least two genes in a genome. Gene fusion events can result in cancer fusion genes, where the aberrant juxtaposition of two or more genes can encode a fusion protein, or the regulatory elements of one gene can drive the aberrant expression of an oncogene. Detecting such cancer fusion genes can be difficult. Breakpoint fragments are less likely to hybridize to probes to the same extent as fragments that do not contain breakpoints. Therefore, hybridization methods for enrichment of breakpoint fragments can lack efficacy.

Fusion genes are a form of somatic mutation found in cancer cells. The ability to detect such fusion genes is useful in the diagnosis and monitoring of cancer.

Fusion genes known to be found in cancer include, for example, the following: APIP/SLC1A2 in colon cancer, ATG7/RAF1 in pancreatic cancer, BCL6/RAF1 in astrocytoma, BCR-ABL in chronic myeloid leukemia, BRD4-NUT in midline carcinomas, CEP85L/ROS1 in angiosarcoma, CLTC/VMP1 in breast cancer, ELM4-ALK in lung cancer, EWSR1/CREM in in melanoma, FAM133B/CDK6 in T-cell acute lymphoblastic leukemia, KIAA1549-BRAF (at 7q34) in low-grade astrocytoma, MECT1-MAML2 in mucoepidermoid carcinoma, PAX8-PPARG in follicular thyroid carcinoma, RET-NTRK1 in papillary thyroid carcinoma, SEC16A-NOTCH1 in breast cancers, SRGAP3-RAF1 (at 3p25) in low-grade astrocytoma, TFE3-TFEB in kidney cancer.

Breakpoints can occur at many different locations in a gene involved in gene fusion. Such breakpoint may be clustered at certain parts of the gene.

One method of detecting gene fusions is by FISH (fluorescent in situ hybridization). Another is by deoxyribonucleic acid (DNA) sequencing.

### SUMMARY

Recognized herein is the need for methods to enrich breakpoint fragments in order to detect and characterize cancer fusion genes.

The present disclosure provides methods to detect fusion genes, which may be used to detect a disease, such as cancer. Provided herein are methods for enrichment of breakpoint fragments, such as to detect and characterize fusion genes, which may be associated with a disease, such as cancer.

In an aspect, the present disclosure provides a method for providing a diagnostic or therapeutic intervention to a subject having or suspected of having cancer, comprising (a) providing a biological sample comprising cell-free nucleic acid molecules from a subject; (b) contacting the cell-free nucleic acid molecules from the biological sample with a probe set under hybridization conditions sufficient to produce probe-captured polynucleotides, which probe set comprises a plurality of polynucleotide probes, wherein each of the plurality of polynucleotide probes has (i) sequence complementarity with a fusion gene and (ii) affinity for the fusion gene that is greater than a polynucleotide having sequence complementary with the fusion gene and containing only unmodified nucleotides; (c) isolating the probe-captured polynucleotides from the mixture, to produce a sample enriched with isolated polynucleotides comprising breakpoint fragments of the fusion gene; (d) sequencing the isolated polynucleotides to produce sequences; (e) detecting polynucleotides comprising breakpoints of fusion genes based on the sequences; and (f) providing the diagnostic or therapeutic intervention based on the detection of breakpoint fragments.

In some embodiments, each of the plurality of polynucleotide probes comprises one or more locked nucleic acid (LNA) nucleotides. In some embodiments, each of the plurality of polynucleotide probes comprises a plurality LNA nucleotides, wherein at least two of the LNA nucleotides are spaced no more than 30 nucleotides apart. In some embodiments, the at least two of the LNA nucleotides are spaced no more than 15 apart.

In some embodiments, at least 50% of the nucleotides of each of at least a subset of the plurality of polynucleotide probes are locked nucleic acid (LNA) nucleotides. In some embodiments, at least 75% of the nucleotides of each of at least a subset of the plurality of polynucleotide probes are locked nucleic acid (LNA) nucleotides.

In some embodiments, each of the plurality of polynucleotide probes has a melting temperature that is at least about 1 °C higher than the polynucleotide having sequence complementary with the fusion gene and containing only unmodified nucleotides. In some embodiments, the melting temperature is at least about 10 °C higher.

In some embodiments, each of the plurality of polynucleotide probes has a melting temperature that is at least about 2 % higher than the polynucleotide having sequence complementary with the fusion gene and containing only unmodified nucleotides. In some embodiments, the melting temperature is at least about 10% higher.

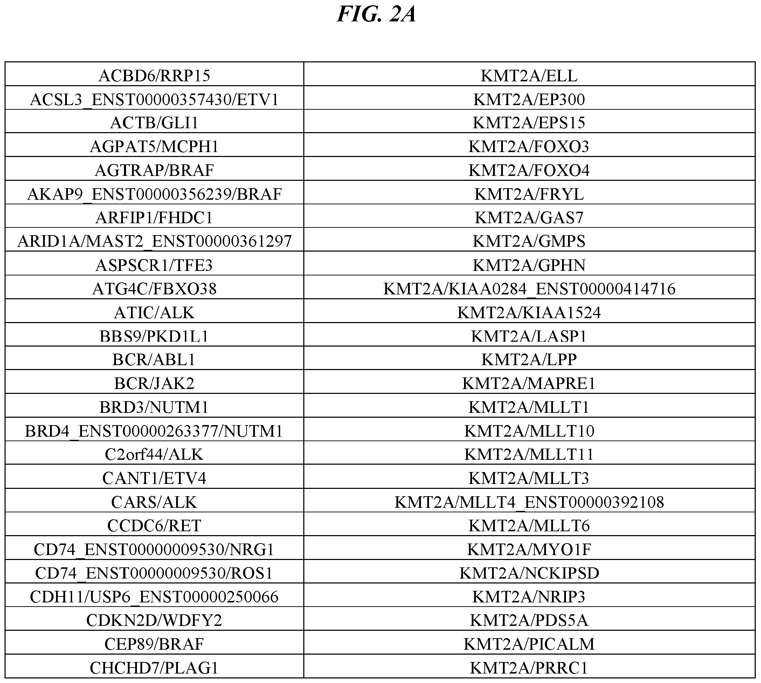

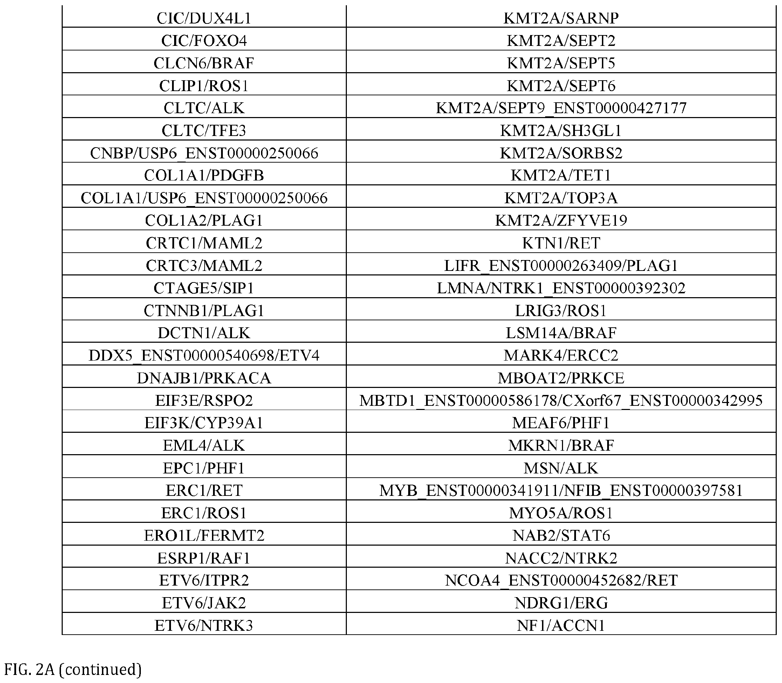

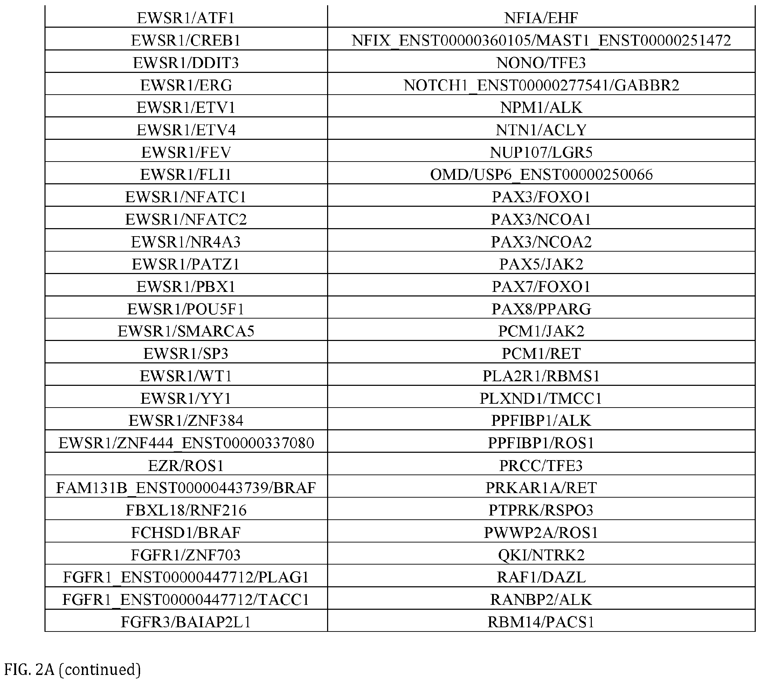

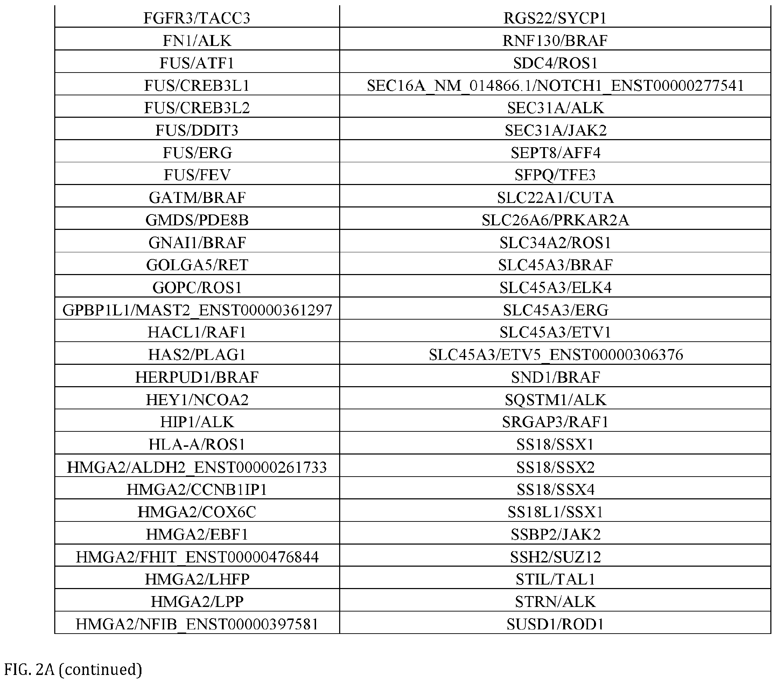

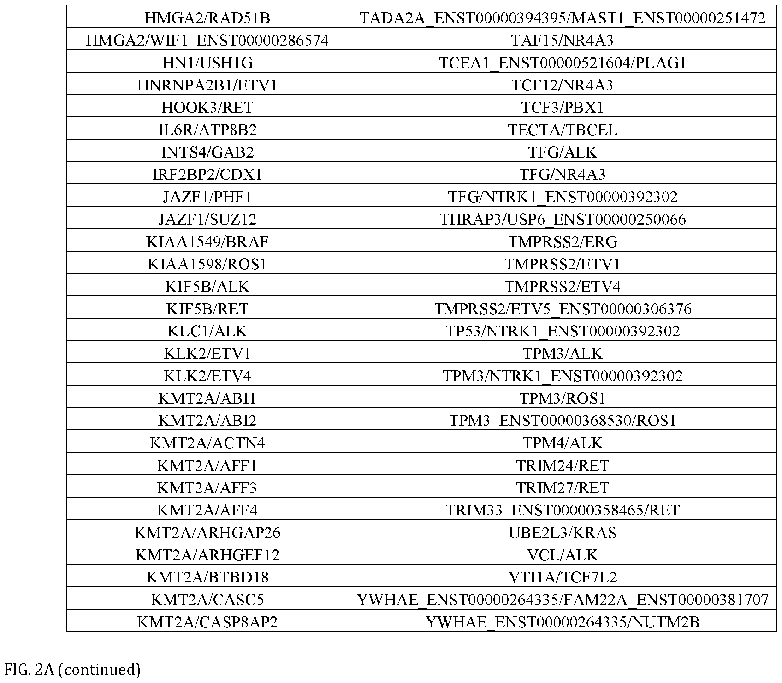

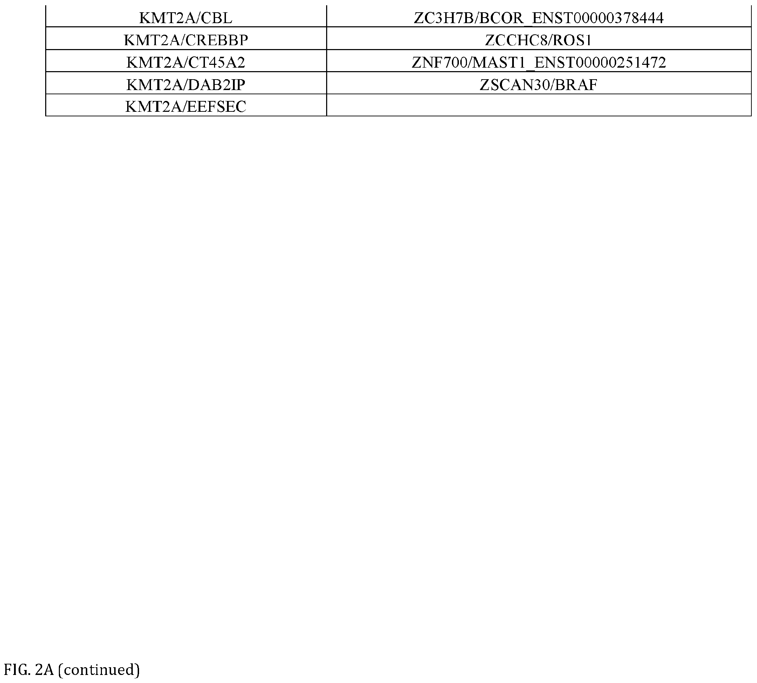

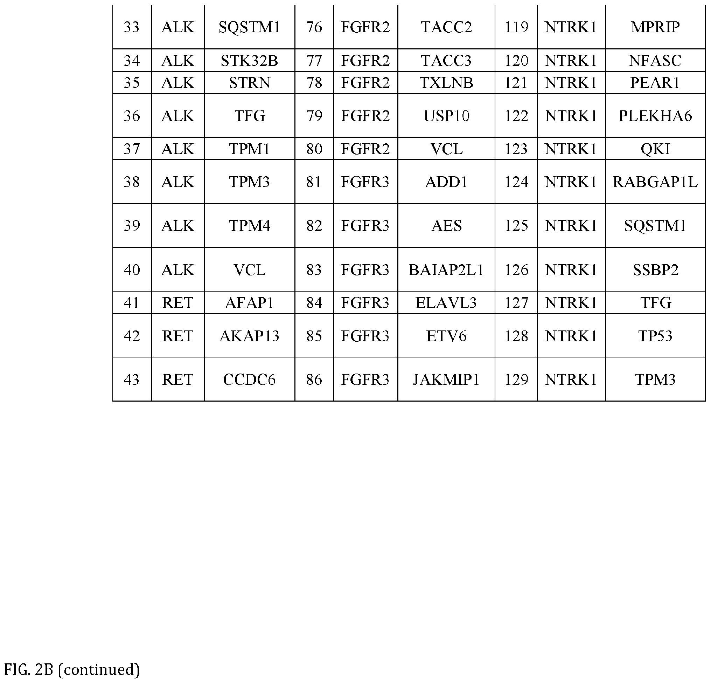

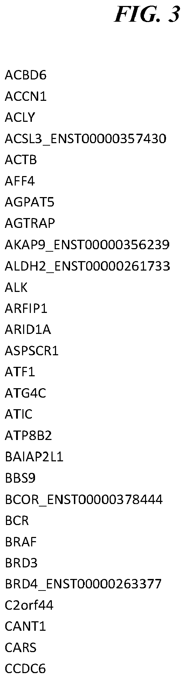

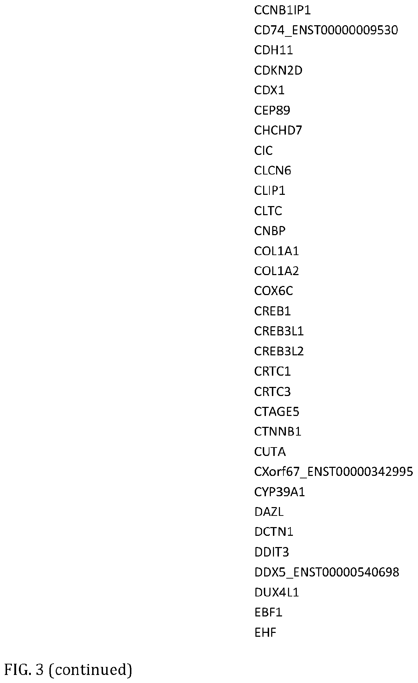

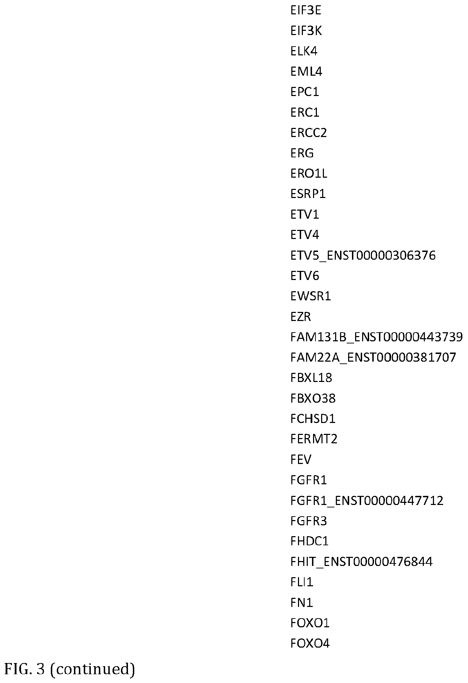

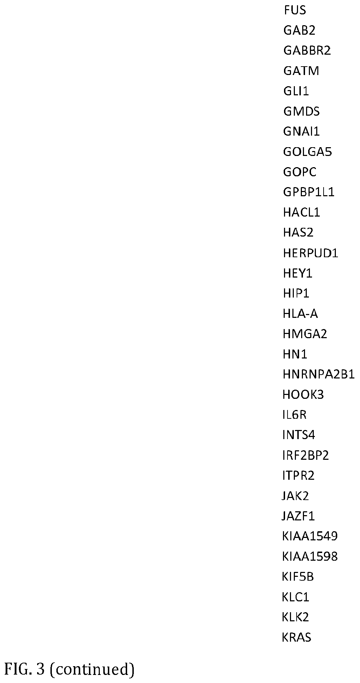

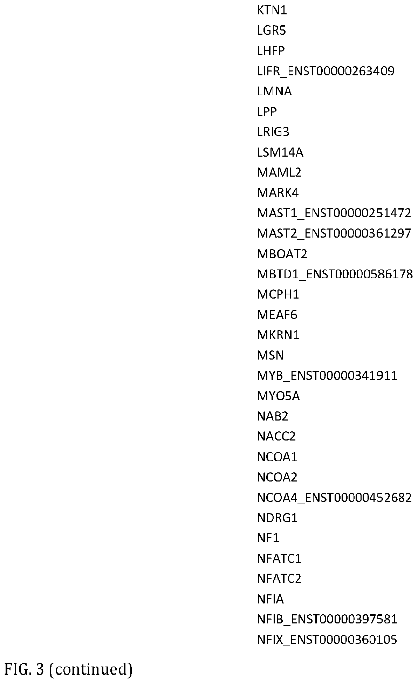

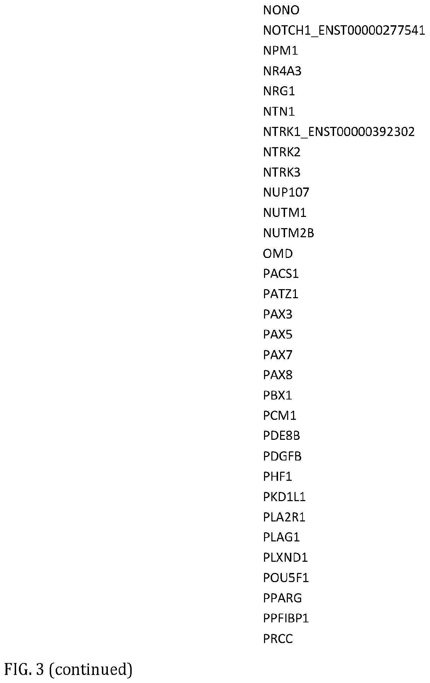

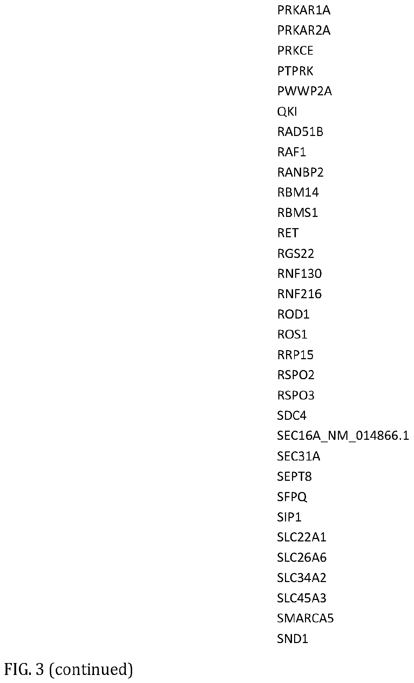

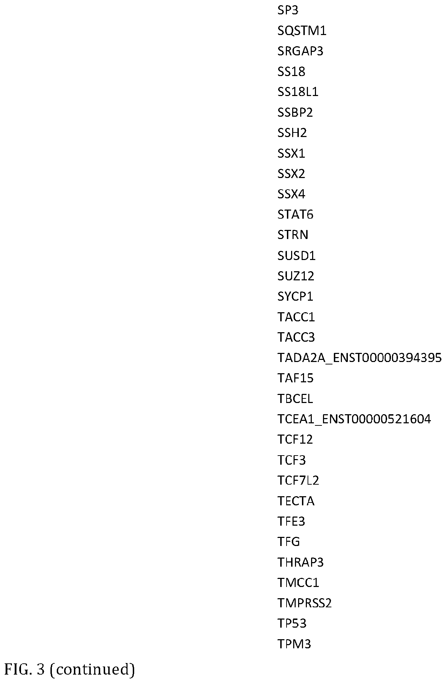

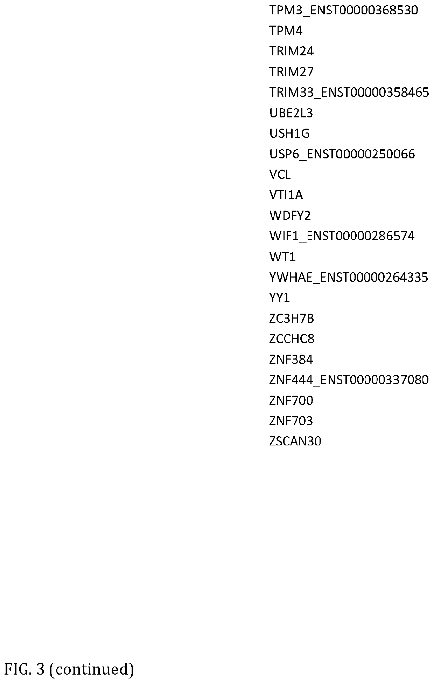

In some embodiments, the fusion gene is a cancer fusion gene. In some embodiments, each of the plurality of polynucleotide probes has sequence complementarity with a gene of a fusion gene pair of FIGs. 2A-2B or a fusion gene between two or more genes selected from FIG. 3. In some embodiments, each of the plurality of polynucleotide probes has sequence complementarity with a breakpoint region no more than 500 nucleotides away from a breakpoint of the fusion gene. In some embodiments, each of the plurality of polynucleotide probes has sequence complementarity with a sequence across a breakpoint in the fusion gene.

In some embodiments, each of the plurality of polynucleotide probes has a length less than about 500 nucleotides. In some embodiments, each of the plurality of polynucleotide probes has a length between about 20 and about 200 nucleotides. In some embodiments, each of the plurality of polynucleotide probes has a length between about 80 and about 160 nucleotides.

In some embodiments, each of the breakpoint fragments has a length between about 140 nucleotides and 180 nucleotides.

In some embodiments, the plurality of polynucleotide probes is coupled to a solid support. In some embodiments, the probe set comprises one or more natural polynucleotide probes. In some embodiments, the plurality of polynucleotide probes comprises at least one polynucleotide probe that hybridizes to a breakpoint region of a nucleic acid sequence included in the fusion gene, and at least one natural polynucleotide probe that hybridizes to a non-breakpoint region of the nucleic acid sequence included in the fusion gene.

In some embodiments, each of the plurality of polynucleotide probes provides at least 50% coverage of a breakpoint region of a nucleic acid sequence included in the fusion gene.

In some embodiments, (d) comprises attaching, to the isolated polynucleotides, tags comprising barcodes having distinct barcode sequences to generate tagged parent polynucleotides. In some embodiments, the method further comprises amplifying the tagged parent polynucleotides to produce tagged progeny polynucleotides.

In some embodiments, the method further comprises (i) sequencing the tagged progeny polynucleotides to produce sequence reads, wherein each sequence read comprises a barcode sequence and a sequence derived from a given one of the isolated polynucleotides, and (ii) grouping the sequence reads into families based at least on the barcode sequence.

In some embodiments, the method further comprises comparing the sequence reads grouped within each family to determine consensus sequences for each family, wherein each of the consensus sequences corresponds to a unique polynucleotide among the tagged parent polynucleotides.

In another aspect, the present disclosure provides a method for capturing a breakpoint fragment of a fusion gene, comprising (a) providing a biological sample containing or suspected of containing a cell-free nucleic acid molecule comprising the breakpoint fragment of the fusion gene; and (b) contacting the biological sample with a polynucleotide probe under conditions sufficient to (i) permit hybridization between the polynucleotide probe and the breakpoint fragment to provide a probe-captured polynucleotide in a mixture, which polynucleotide probe has sequence complementarity with the breakpoint fragment and has affinity for the fusion gene that is greater than a polynucleotide having sequence complementary with the fusion gene and containing only unmodified nucleotides; and (ii) enrichment or isolation of the probe-captured polynucleotide from the mixture, wherein the polynucleotide probe has sequence complementarity with the breakpoint fragment.

In some embodiments, the polynucleotide probe comprises one or more locked nucleic acid (LNA) nucleotides. In some embodiments, the polynucleotide probe comprises a plurality LNA nucleotides, wherein at least two of the LNA nucleotides are spaced no more than 30 nucleotides apart. In some embodiments, the at least two of the LNA nucleotides are spaced no more than 15 nucleotides apart.

Another aspect of the present disclosure provides a probe set comprising a plurality of polynucleotide probes, wherein each of the polynucleotide probes has (i) sequence complementarity with a fusion gene as part of a cell-free nucleic acid molecule and (ii) affinity for the fusion gene that is greater than a polynucleotide having sequence complementary with the fusion gene and containing only unmodified nucleotides.

In some embodiments, each of the plurality of polynucleotide probes comprises one or more locked nucleic acid nucleotides. In some embodiments, the probe set further comprises one or more natural polynucleotide probes. In some embodiments, each of the plurality of polynucleotide probes comprises at least one polynucleotide probe that hybridizes to a breakpoint region of a nucleic acid sequence included in the fusion gene, and at least one natural polynucleotide probe that hybridizes to a non-breakpoint region of the nucleic acid sequence included in the fusion gene.

In some embodiments, each of the plurality of polynucleotide probes provides at least 50% coverage of a breakpoint region of a nucleic acid sequence included in the fusion gene.

In some embodiments, the plurality of polynucleotide probes hybridize to portions of one or both of the different genes in the fusion gene.

In some embodiments, the probe set further comprises a solid support, wherein the plurality of polynucleotide probes is coupled to the solid support.

In some embodiments, each of the plurality of polynucleotide probes has a melting temperature that is at least about 1 °C higher than the polynucleotide having sequence complementary with the fusion gene and containing only unmodified nucleotides. In some embodiments, the melting temperature is at least about 10 °C higher.

In some embodiments, each of the plurality of polynucleotide probes has a melting temperature that is at least about 2 % higher than the polynucleotide having sequence complementary with the fusion gene and containing only unmodified nucleotides. In some embodiments, the melting temperature is at least about 10% higher.

In some embodiments, the fusion gene is a cancer fusion gene.

In some embodiments, each of the plurality of polynucleotide probes has sequence complementarity with a gene of a fusion gene pair of FIGs. 2A-2B or a fusion gene between two or more genes selected from FIG. 3.

In another aspect, disclosed herein is a high affinity polynucleotide, comprising a sequence that is configured to specifically hybridize to a nucleic acid sequence associated with a fusion gene in a cell-free nucleic acid molecule.

In another aspect, disclosed herein is a high affinity polynucleotide configured to specifically hybridize to a fusion gene. In one embodiment the high affinity polynucleotide comprises one or more locked nucleic acid nucleotides. In another embodiment the high affinity polynucleotide has a melting temperature that is at least any of 1 °C, 2 °C, 3 °C, 4 °C, 5 °C, 10 °C, 15 °C or 20 °C higher than a polynucleotide with the same sequence comprising only natural nucleotides. In another embodiment the high affinity polynucleotide has a melting temperature that is at least any of 2%, 4%, 6%, 8%, or 10% higher than a polynucleotide with the same sequence comprising only natural nucleotides. In another embodiment the high affinity polynucleotide is configured to specifically hybridize to a cancer fusion gene. In another embodiment the high affinity polynucleotide is configured to specifically hybridize to a gene of a fusion gene pair of FIGs. 2A-2B or a fusion gene between at least any of 1, 2, 3, 4, 5, 6, 7, 8, 9, 10, or more genes selected from FIG. 3. In another embodiment the high affinity polynucleotide is configured to hybridize within a breakpoint region no more than 500 nucleotides away from a breakpoint of the fusion gene. In another embodiment the high affinity polynucleotide is configured to hybridize across a breakpoint in the fusion gene. In another embodiment the high affinity polynucleotide has a length less than about 500 nucleotides, between about 20 and about 200 nucleotides, or between about 80 and about 160 nucleotides. In another embodiment the high affinity polynucleotide comprises a plurality of locked nucleic acid (LNA) nucleotides, wherein at least two of the LNA nucleotides are spaced no more than 30, 20, 15, 10 or 5 nucleotides apart. In another embodiment 100%, or at least any of 90%, 75%, 50%, 20%, 10%, or 5% or 1% of the nucleotides in the polynucleotide are locked nucleic acid nucleotides. In another embodiment the high affinity polynucleotide is has a nucleotide sequence perfectly or substantially complementary to a nucleotide sequence of the fusion gene.

In another aspect this disclosure provides a high affinity polynucleotide probe comprising a high affinity polynucleotide configured to specifically hybridize to a fusion gene. In one embodiment the high affinity polynucleotide comprises one or more locked nucleic acid nucleotides. In another embodiment the probe comprises a functionality selected from a detectable label, a binding moiety or a solid support. In another embodiment the probe is configured to hybridize to a breakpoint fragment of a fusion gene. In another embodiment the breakpoint fragment has a length between about 140 nucleotides and about 180 nucleotides. In another embodiment the fragment is cell-free deoxyribonucleic acid (DNA) or genomic DNA. In another embodiment the high affinity polynucleotide is bound to a solid support.

In another aspect this disclosure provides a probe set comprising a plurality of polynucleotide probes, each probe configured to specifically hybridize to a fusion gene, wherein the set comprises one or more high affinity polynucleotide probes. In one embodiment the high affinity polynucleotide comprises one or more locked nucleic acid nucleotides. In another embodiment the set comprises one or more natural polynucleotide probes. In another embodiment the probe set comprises at least one high affinity polynucleotide probe that specifically hybridizes to a breakpoint region of a gene involved in the fusion gene, and at least one natural polynucleotide probe that hybridizes to a non-breakpoint region of the gene involved in the fusion gene. In another embodiment the one or more high affinity polynucleotide probes in the probe set provide at least 50% (e.g., at least 0.5X to 5X) coverage of a breakpoint region of a gene involved in the fusion gene. In another embodiment the probes hybridize to portions of one or both of the different genes in the fusion gene. In another embodiment the probe set is configured as an oligonucleotide chip. In another embodiment a target sequence is targeted by both high affinity polynucleotide probes and standard affinity polynucleotide probes.

In another aspect this disclosure provides a kit comprising a plurality of probe sets, wherein each probe set specifically hybridizes to a different gene and at least one of the probe sets is a probe set of this disclosure. In one embodiment the high affinity polynucleotide comprises one or more locked nucleic acid nucleotides.

In another aspect, this disclosure provides a method for capturing a breakpoint fragment of a fusion gene comprising contacting the breakpoint fragment with a high affinity polynucleotide probe under stringent hybridization conditions and allowing hybridization, wherein the polynucleotide probe is bound to a solid support and wherein the polynucleotide probe has a nucleotide sequence that is substantially or perfectly complementary to a nucleotide sequence of the breakpoint fragment. In one embodiment the high affinity polynucleotide comprises one or more locked nucleic acid nucleotides.

In another aspect, this disclosure provides a method for enriching a sample for polynucleotides comprising a breakpoint of a fusion gene, comprising: a) contacting a probe set of claim 20 with a mixture of polynucleotides under hybridization conditions to produce probe-captured polynucleotides; and b) isolating the probe-captured polynucleotides from the mixture, to produce a sample enriched with polynucleotides comprising breakpoint fragments of the fusion gene. In one embodiment the high affinity polynucleotide comprises one or more locked nucleic acid nucleotides. In another embodiment the polynucleotides comprise cell-free DNA or fragmented genomic DNA. In another embodiment the method further comprises isolating captured polynucleotides from the probes. In another embodiment the method further comprises sequencing the isolated polynucleotides.

In another aspect, this disclosure provides method of diagnosing cancer in a subject comprising: a) providing a sample comprising polynucleotides from a subject; b) contacting the cell-free DNA (cfDNA) from the sample with a probe set of claim 20 under hybridization conditions to produce probe-captured polynucleotides; c) isolating the probe-captured polynucleotides from the mixture, to produce a sample enriched with polynucleotides comprising breakpoint fragments of the fusion gene; d) sequencing the isolated polynucleotides to produce sequences; e) detecting polynucleotides comprising breakpoints of fusion genes based on the sequences; and f) diagnosing cancer based on the detection of breakpoint fragments. In one embodiment the high affinity polynucleotide comprises one or more locked nucleic acid nucleotides.

Another aspect of the present disclosure provides a non-transitory computer-readable medium comprising machine executable code that, upon execution by one or more computer processors, implements any of the methods above or elsewhere herein.

Another aspect of the present disclosure provides a system comprising one or more computer processors and a non-transitory computer-readable medium coupled thereto. The non-transitory computer readable medium comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG. 1** depicts breakpoint fragments derived from a fusion gene and the loss of such fragments during standard probe capture protocols;
**FIG. 2A** provides a list of cancer fusion gene pairs; **FIG. 2B** provides another list of cancer fusion gene pairs;
**FIG. 3** provides a list of genes detected in cancer fusion genes;
**FIGS. 4A-4U** provide exemplary breakpoints for cancer fusion gene pairs;
**FIGS. 5A-B** shows different coverage depths and tiling for probes and/or polynucleotides;
**FIGS. 6A-6D** shows different exemplary mixtures of high affinity probe sequence subsets and standard affinity probe sequence subsets;
**FIG. 7** shows a 64 gene panel, including four genes, ALK, NKRT1, RET and ROS1, involved in gene rearrangements;
**FIG. 8** shows eight genomic regions of the ALK gene that may be targeted for deeper coverage; and
**FIG. 9** shows a computer control system that is programmed or otherwise configured to implement methods provided herein.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

### I. Definitions

"High affinity polynucleotide", as used herein, refers to a polynucleotide comprising at least one chemical modification that provides the polynucleotide with a higher melting temperature in a hybridization reaction compared with a same sequence polynucleotide not so modified. In embodiments, the higher melting temperature can be at least any of 1°, 2°, 3°, 4°, 5°, 10°, 15° or 20° C higher. The polynucleotide can comprise one or more nucleotide analogs, an LNA nucleotide.

"Locked nucleic acid" ("LNA") (sometimes referred to as "inaccessible RNA"), as used herein, refers to a high affinity polynucleotide comprising at least one locked nucleic acid (LNA) nucleotide.

"Locked nucleic acid nucleotide" ("LNA nucleotide") as used herein, refers to a modified RNA nucleotide that provides the polynucleotide with greater thermodynamic stability during hybridization as compared with a polynucleotide that differs from the LNA only by having a natural ribonucleotide in place of the modified RNA nucleotide. In certain embodiments, the ribose moiety of a modified RNA nucleotide is modified with an extra bridge connecting the 2' oxygen and 4' carbon.

LNA nucleotides can comprise any type of extra bridge between the 2'O and 4'C of the RNA that increases the thermodynamic stability of the duplex between the LNA and its complement. In some cases, BNA, the 2' oxygen and 4' carbon are bridged by a methylene group. In some cases, 2'-O,4'-C-ethylene-bridged nucleic acids (ENA), the 2' oxygen and 4' carbon are bridged by an ethylene group. Other examples of BNA can include, but are not limited to, 2',4'-BNA^{NC}[NH], 2',4'-BNA^{NC}[NMe], and 2',4'-BNA^{NC}[NBn].

"Bridged nucleic acid" ("BNA") refers to 2'-O,4'-C-methylene-modified nucleic acids.

Other 2'O-modified nucleotides, such as 2'O-Me, demonstrate greater stability, as well.

"Fusion gene", as used herein, refers to a gene that results from a chromosomal rearrangement (inversion, deletion, translocation) that brings together formerly separate portions of at least two different genes in a genome.

"Cancer fusion gene", as used herein, refers to a fusion gene resulting from somatic mutation in a cancer cell.

"Breakpoint", as used herein, refers to a nucleotide position in a fusion gene at which portions of two different genes are fused.

"Breakpoint region", as used herein, refers to a region of a gene that can be involved in gene fusions at which a breakpoint can occur.

"Breakpoint fragment" of a fusion gene, as used herein, refers to a fragment of a fusion gene that includes sequences from two different genes making up the fusion gene.

"Probe", as used herein, refers to a polynucleotide comprising a functionality. The functionality can be a detectable label (fluorescent), a binding moiety (biotin), or a solid support (a magnetically attractable particle or a chip).

"Natural polynucleotide" or "natural oligonucleotide", as used herein, refers to a polynucleotide or an oligonucleotide in which all of the nucleotides in the probe are natural nucleotides.

"Complementarity" refers to the ability of a nucleic acid to form hydrogen bond(s) with another nucleic acid sequence by either traditional Watson-Crick or other non-traditional types. A percent complementarity indicates the percentage of residues in a nucleic acid molecule which can form hydrogen bonds (Watson-Crick base pairing) with a second nucleic acid sequence (5, 6, 7, 8, 9, 10 out of 10 being 50%, 60%, 70%, 80%, 90%, and 100% complementary, respectively). "Perfectly complementary" means that all the contiguous residues of a nucleic acid sequence will hydrogen bond with the same number of contiguous residues in a second nucleic acid sequence.

"Substantially complementary" as used herein refers to a degree of complementarity that is at least any of 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions. Sequence identity, such as for the purpose of assessing percent complementarity, may be measured by any suitable alignment algorithm, including but not limited to the Needleman-Wunsch algorithm (see e.g. the EMBOSS Needle aligner available at the world wide web site: ebi.ac.uk/Tools/psa/emboss_needle/nucleotide.html, optionally with default settings), the BLAST algorithm (see e.g. the BLAST alignment tool available at blast.ncbi.nlm.nih.gov/Blast.cgi, optionally with default settings), or the Smith-Waterman algorithm (see e.g. the EMBOSS Water aligner available at the world wide web site: ebi.ac.uk/Tools/psa/emboss_water/nucleotide.html, optionally with default settings). Optimal alignment may be assessed using any suitable parameters of a chosen algorithm, including default parameters.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner according to base complementarity. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the enzymatic cleavage of a polynucleotide by an endonuclease. A second sequence that is complementary to a first sequence is referred to as the "complement" of the first sequence. The term "hybridizable" as applied to a polynucleotide refers to the ability of the polynucleotide to form a complex that is stabilized via hydrogen bonding between the bases of the nucleotide residues in a hybridization reaction.

"Specifically hybridize to" or "hybridizing specifically to" or "specific hybridization" refers to the formation of a stable duplex between two polynucleotides under conditions of 50% formamide, 5 x SSC and 1% SDS incubated at 42° C or 5 x SSC and 1% SDS incubated at 65° C, with a wash in 0.2 x SSC and 0.1% SDS at 65° C.

The term "stringent hybridization conditions" refers to conditions under which a polynucleotide will hybridize preferentially to its target subsequence, and to a lesser extent to, or not at all to, other sequences. "Stringent hybridization" in the context of nucleic acid hybridization experiments are sequence dependent, and are different under different environmental parameters. An extensive guide to the hybridization of nucleic acids is found in Tijssen (1993) Laboratory Techniques in Biochemistry and Molecular Biology--Hybridization with Nucleic Acid Probes part I chapter 2 "Overview of principles of hybridization and the strategy of nucleic acid probe assays", Elsevier, New York.

Generally, highly stringent hybridization and wash conditions are selected to be about 5° C lower than the thermal melting point (Tm) for the specific sequence at a defined ionic strength and pH. The Tm is the temperature (under defined ionic strength and pH) at which 50% of the target sequence hybridizes to a perfectly matched probe. Very stringent conditions are selected to be equal to the Tm for a particular probe.

Stringent hybridization conditions include a buffer comprising water, a buffer (a phosphate, tris, SSPE or SSC buffer at pH 6-9 or pH 7-8), a salt (sodium or potassium), and a denaturant (SDS, formamide or tween) and a temperature of 37° C -70° C, 60° C -65° C.

An example of stringent hybridization conditions for hybridization of complementary nucleic acids which have more than 100 complementary residues on a filter in a Southern or northern blot is 50% formalin with 1 mg of heparin at 42° C, with the hybridization being carried out overnight. An example of highly stringent wash conditions is 0.15 M NaCI at 72° C for about 15 minutes. An example of stringent wash conditions is a 0.2X SSC wash at 65° C for 15 minutes (see, Sambrook et al. for a description of SSC buffer). Often, a high stringency wash is preceded by a low stringency wash to remove background probe signal. An example medium stringency wash for a duplex of, more than 100 nucleotides, is 1x SSC at 45° C for 15 minutes. An example low stringency wash for a duplex of, e. g., more than 100 nucleotides, is 4-6x SSC at 40° C for 15 minutes. In general, a signal to noise ratio of 2x (or higher) than that observed for an unrelated probe in the particular hybridization assay indicates detection of a specific hybridization.

### II. Overview

Provided herein are compositions and methods for detecting polynucleotides comprising one or more fusion genes. The polynucleotides can be deoxyribonucleic acid (DNA). The compositions and methods provided herein can detect fusion genes with high sensitivity in heterogeneous polynucleotide samples, such as cell-free DNA ("cfDNA").

DNA from cells, including cancer cells, can be shed into the blood in the form of cell-free DNA. Cell-free DNA has an average length of about 160 nucleotides. Because fragmentation does not occur at pre-specified points, for any genomic locus, fragments may be found in a sample that tile across that locus.

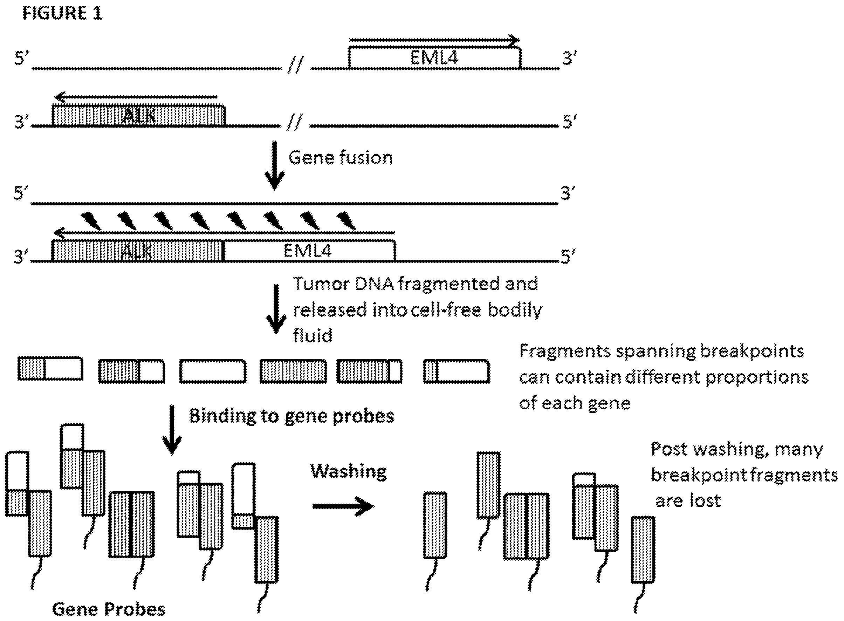

In cancer, certain genes are commonly involved in gene fusions with other genes. For example, the EML4 and ALK genes commonly undergo gene fusion with each other in cancer. The breakpoint of each gene involved in a fusion can occur at breakpoint regions ("hot spots") in each of the genes. When cells containing these fusion genes die, their DNA is shed into the blood in the form of cfDNA. As shown in FIG. 1, the position in the fragment mapping to the breakpoint may occur anywhere in the fragment, near the 5' end, in the middle, or near the 3' end. Accordingly, the cfDNA polynucleotide can have a very short or a very long nucleotide sequence from either gene involved in the fusion.

Certain DNA sequencing methods use sequence capture to enrich for sequences of interest. Sequence capture typically involves the use of oligonucleotide probes that hybridize to the sequence of interest. The probe set strategy can involve tiling the probes across a region of interest. Such probes can be, about 120 bases long. The set can have a depth of about 2x. The effectiveness of sequence capture depends, in part, on the length of the sequence in the target molecule that is complementary (or nearly complementary) to the sequence of the probe.

However, in the case of fusion genes, polynucleotides mapping to the breakpoint may contain a sequence from the target gene that is shorter than optimal for hybridization and capture. For example, a cfDNA fragment mapping to a fusion involving an ALK-EML4 fusion may have, for example, a 150 nucleotide sequence of the ALK gene, a 100 nucleotide sequence, a 50 nucleotide sequence, a 25 nucleotide sequence or a 10 nucleotide sequence. In this case, there is a lower probability of capturing the polynucleotide if it has a shorter ALK sequence than of capturing a polynucleotide with a sequence fully complementary to the ALK probe. The problem is more acute when sequence capture is multiplex, targeting sequences from many different genes.

Provided herein are materials and methods for capturing polynucleotide fragments mapping to a breakpoint in a fusion gene. Such polynucleotides are captured using high affinity polynucleotide probes, such locked nucleic acids. Such probes have higher melting temperature than probes of the same sequence made from natural nucleotides. Consequently, they produce higher yield of captured products from the same sample.

Such probes can be included in a probe set targeting both fusion genes and non-fused genes. In this way, captured polynucleotides are enriched for those including fusion genes, compared with a population captured using only probes made from natural nucleotides.

An exemplary probe set can contain, for example, a subset of LNA probes. The LNA probes can be configured to tile across breakpoint regions of genes involved in fusion genes.

Every nucleotide in an LNA probe can be an LNA nucleotide. Alternatively, a fraction of the nucleotides can be LNA nucleotides. In certain embodiments, the LNA nucleotides can be spaced a predetermined number of nucleotides apart.

The present invention provides high-affinity polynucleotides that can be used to enrich a sample containing nucleic acid fragments for those nucleic acid fragments that contain gene fusion events. These high-affinity polynucleotides can contain LNA nucleotides. Substituting LNA nucleotides for standard nucleotides can increase the melting temperature of the high-affinity polynucleotide, thereby increasing the stability of the duplex between the high-affinity polynucleotide and a nucleic acid fragment that contains a fusion gene.

Gene fusions can be associated with, and in some cases contribute to, the development of a healthy cell into a neoplasm (a tumor or an adenoma). Detecting these gene fusion events may provide a useful approach for detecting and/or monitoring the presence of a neoplasm in a patient. Breakpoint fragments, however, will have less sequence derived from either gene flanking the breakpoint than a nucleic acid fragment of a similar length comprising sequence from just one of the genes. For this reason, a breakpoint fragment is often only capable of binding to a reduced section of a gene probe or gene-specific oligonucleotide. If the hybridization and wash conditions have been optimized for full-length or near full-length binding, the nucleic acid fragment containing the breakpoint can hybridize with insufficient affinity and be lost (see FIG. 1). Furthermore, in a heterogeneous sample containing nucleic acid fragments from cells that have and have not undergone gene fusion events, nucleic acid fragments from those that have not undergone gene fusion events can bind more stably to the gene probe or gene-specific oligonucleotide and competitively inhibit the hybridization of nucleic acid fragments containing breakpoints.

Tumor-derived nucleic acid can be found in cell-free bodily fluids. Tumor-derived nucleic acids from such cell-free bodily fluids can be assayed for nucleic acid fragments containing fusion genes in order to detect neoplasms. Cell-free bodily fluids can contain small amounts of tumor-derived nucleic acid, and the tumor-derived nucleic acid can be admixed with nucleic acid that is derived from healthy tissue. The present disclosure also provides approaches for enriching for nucleic acid fragments that contain fusion genes from nucleic acid derived from a cell-free bodily fluid.

### III. Test Samples

### A. Subject Types

Samples are collected from subjects, e.g. patients at risk for developing cancer. The subjects may be patients with no known risk factors for cancer. The subjects can be patients whose only risk factors for cancer are age and/or gender. In some cases, the subjects can have known risk factors for cancer, e.g. smoking or familial history of cancer. In some cases, the subjects can be patients with symptoms of cancer.

Other subjects can be patients with neoplasms that have previously been detected, by colonoscopy or imaging. The samples derived from patients with previously detected neoplasms can be assayed for nucleic acid fragments containing breakpoints in order to recommend a course of treatment or therapy. The samples derived from patients with neoplasms can be assayed for nucleic acids fragments containing breakpoints in order to determine the effectiveness of the treatment or therapy they are receiving.

Other subjects can be patients with neoplasms that have been previously detected, but in whom the neoplasm is no longer detectable (patients in remission or who have no evidence of disease). The samples derived from patients in whom the neoplasm is no longer detectable can be assayed for nucleic acid fragments containing breakpoints in order to detect a relapse or reemergence of the neoplasm.

Other subjects can be women with a familial history of cancer, wherein the genetic defect responsible for the familial cancer is known or suspected to be a fusion gene. In some cases, a woman with a family history of cancer may be pregnant and want to determine whether the fetus she is carrying has the fusion gene. In some cases, a sample containing fetal nucleic acids from such a subject can be assayed for the gene fusion event.

### B. Sample Types

Samples can be nucleic acids extracted from various sources. Nucleic acids can be, but are not limited to, genomic DNA, RNA, mitochondrial DNA, fetal DNA, and miRNA.

Samples may be extracted from a variety of bodily fluids containing cell-free nucleic acids, including but not limited to blood, serum, plasma, vitreous, sputum, urine, tears, perspiration, saliva, semen, mucosal excretions, mucus, spinal fluid, amniotic fluid, lymph fluid and the like. The collection of bodily fluids can be achieved using a variety of techniques. In some cases, collection may comprise aspiration of a bodily fluid from a subject using a syringe. In other cases collection may comprise pipetting or direct collection of fluid into a collecting vessel.

After collection of bodily fluid, cell-free nucleic acids may be isolated and extracted using a variety of techniques. In some cases, cell-free nucleic acids may be isolated, extracted and prepared using commercially available kits such as the Qiagen Qiamp® Circulating Nucleic Acid Kit protocol. In other examples, Qiagen Qubit™ dsDNA HS Assay kit protocol, Agilent™ DNA 1000 kit, or TruSeq™ Sequencing Library Preparation; Low-Throughput (LT) protocol may be used to quantify nucleic acids. Cell-free nucleic acids may be fetal in origin (via fluid taken from a pregnant subject), or may be derived from tissue of the subject itself. Cell-free nucleic acids can be derived from a neoplasm (e.g. a tumor or an adenoma).

Generally, cell-free nucleic acids are extracted and isolated from bodily fluids through a partitioning step in which cell-free nucleic acids, as found in solution, are separated from cells and other non-soluble components of the bodily fluid. Partitioning may include, but is not limited to, techniques such as centrifugation or filtration. In other cases, cells are not partitioned from cell-free nucleic acids first, but rather lysed. In one example, the genomic DNA of intact cells is partitioned through selective precipitation. Cell-free nucleic acids, including DNA, may remain soluble and may be separated from insoluble genomic DNA and extracted. Generally, after addition of buffers and other wash steps specific to different kits, nucleic acids may be precipitated using isopropanol precipitation. Further clean up steps may be used such as silica based columns to remove contaminants or salts. General steps may be optimized for specific applications. Non-specific bulk carrier nucleic acids, for example, may be added throughout the reaction to optimize certain aspects of the procedure such as yield.

Cell-free nucleic acids can be at most 500 nucleotides in length, at most 400 nucleotides in length, at most 300 nucleotides in length, at most 250 nucleotides in length, at most 225 nucleotides in length, at most 200 nucleotides in length, at most 190 nucleotides in length, at most 180 nucleotides in length, at most 170 nucleotides in length, at most 160 nucleotides in length, at most 150 nucleotides in length, at most 140 nucleotides in length, at most 130 nucleotides in length, at most 120 nucleotides in length, at most 110 nucleotides in length, or at most 100 nucleotides in length.

Cell-free nucleic acids can be at least 500 nucleotides in length, at least 400 nucleotides in length, at least 300 nucleotides in length, at least 250 nucleotides in length, at least 225 nucleotides in length, at least 200 nucleotides in length, at least 190 nucleotides in length, at least 180 nucleotides in length, at least 170 nucleotides in length, at least 160 nucleotides in length, at least 150 nucleotides in length, at least 140 nucleotides in length, at least 130 nucleotides in length, at least 120 nucleotides in length, at least 110 nucleotides in length, or at least 100 nucleotides in length. In particular, cell-free nucleic acids can be between 140 and 180 nucleotides in length.

A sample may be extracted from tissue from the subject. A sample can be a tumor biopsy. The tumor biopsy can contain a mixture of tumor and healthy tissue. The tumor biopsy can be formaldehyde-fixed and paraffin-embedded. The tumor can be at least 0.1% of the biopsy, at least 0.2% of the biopsy, at least 0.5% of the biopsy, at least 0.7% of the biopsy, at least 1% of the biopsy, at least 2% of the biopsy, at least 3% of the biopsy, at least 4% of the biopsy, at least 5% of the biopsy, at least 10% of the biopsy, at least 15% of the biopsy, at least 20% of the biopsy, at least 25% of the biopsy, or at least 30% of the biopsy. A sample can be a biopsy from healthy tissue.

Nucleic acids extracted from tissue can be at most 10 kb in length, at most 7 kb in length, at most 5 kb in length, at most 4 kb in length, at most 3 kb in length, at most 2 kb in length, at most 1 kb in length, at most 500 nucleotides in length, at most 400 nucleotides in length, at most 300 nucleotides in length, at most 250 nucleotides in length, at most 225 nucleotides in length, at most 200 nucleotides in length, at most 190 nucleotides in length, at most 180 nucleotides in length, at most 170 nucleotides in length, at most 160 nucleotides in length, at most 150 nucleotides in length, at most 140 nucleotides in length, at most 130 nucleotides in length, at most 120 nucleotides in length, at most 110 nucleotides in length, or at most 100 nucleotides in length.

Nucleic acids extracted from tissue can be at least 5 kb in length, at least 4 kb in length, at least 3 kb in length, at least 2 kb in length, at least 1 kb in length, at least 500 nucleotides in length, at least 400 nucleotides in length, at least 300 nucleotides in length, at least 250 nucleotides in length, at least 225 nucleotides in length, at least 200 nucleotides in length, at least 190 nucleotides in length, at least 180 nucleotides in length, at least 170 nucleotides in length, at least 160 nucleotides in length, at least 150 nucleotides in length, at least 140 nucleotides in length, at least 130 nucleotides in length, at least 120 nucleotides in length, at least 110 nucleotides in length, or at least 100 nucleotides in length.

In some cases, nucleic acids can be sheared during the extraction process and comprise fragments between 100 and 400 nucleotides in length. In some cases, nucleic acids can be sheared after extraction can comprise nucleotides between 100 and 400 nucleotides in length.

Isolation and purification of cell-free and tissue-derived nucleic acids may be accomplished using various approaches, including, but not limited to, the use of commercial kits and protocols provided by companies such as Sigma Aldrich, Life Technologies, Promega, Affymetrix, IBI or the like. Kits and protocols may also be non-commercially available.

### IV. Genetic Analysis

Genetic analysis includes detection of nucleotide sequence variants, copy number variations, and fusion genes. Genetic variants can be determined by sequencing. The sequencing method can be massively parallel sequencing, that is, simultaneously (or in rapid succession) sequencing any of at least 100,000, 1 million, 10 million, 100 million, or 1 billion polynucleotide molecules. Sequencing methods may include, but are not limited to: high-throughput sequencing, pyrosequencing, sequencing-by-synthesis, single-molecule sequencing, nanopore sequencing, semiconductor sequencing, sequencing-by-ligation, sequencing-by-hybridization, RNA-Seq (Illumina), Digital Gene Expression (Helicos), Next-generation sequencing, Single Molecule Sequencing by Synthesis (SMSS)(Helicos), massively-parallel sequencing, Clonal Single Molecule Array (Solexa), shotgun sequencing, Maxam-Gilbert or Sanger sequencing, primer walking, sequencing using PacBio, SOLiD, Ion Torrent, nanopore-based platforms or other sequencing methods.

Sequencing can be made more efficient by performing sequence capture, that is, the enrichment of a sample for target sequences of interest, sequences of cancer fusion genes and cancer fusion gene breakpoints as described herein. Sequence capture can be performed using immobilized probes that hybridize to the targets of interest. Sequence capture can be performed using probes attached to functional groups, biotin, that allow probes hybridized to specific sequences to be enriched for from a sample by pulldown. In some cases, prior to hybridization to functionalized probes, specific sequences such as adapter sequences from library fragments can be masked by annealing complementary, non-functionalized polynucleotide sequences to the fragments in order to reduce non-specific or off-target binding.

In some cases the cell-free nucleic acid fragments or tissue-derived nucleic acid fragments are inputs to produce sequencing libraries. In some cases, the fragments are enriched for specific sequence prior to preparing a sequencing library. The enriched fragmented nucleic acids can be attached to any sequencing adaptor suitable for use on any sequencing platform disclosed herein. For example, a sequence adaptor can comprise a flow cell sequence, a sample barcode, or both. In another example, a sequence adaptor can be a hairpin shaped adaptor and/or comprise a sample barcode. Further, the resulting fragments can be amplified and sequenced. In some cases, the adaptor does not comprise a sequencing primer region. In some cases, the sequencing libraries are enriched for specific sequences prior to sequencing.

Cell-free nucleic acids can include small amounts of tumor nucleic acids mixed with germline nucleic acids. In some cases, tumor biopsies can include small amounts of tumor tissue mixed in with healthy tissue, and nucleic acids extracted from such samples without enrichment can include small amounts of tumor nucleic acids mixed with germline nucleic acids. Sequencing methods that increase sensitivity and specificity of detecting tumor nucleic acids, and, in particular, genetic sequence variants and copy number variation, can be useful in the methods of this invention. Such methods are described in, for example, in WO 2014/039556, WO 2014/149134 and WO 2015/100427, each of which is entirely incorporated herein by reference. These methods not only can detect molecules with a sensitivity of up to or greater than 0.1%, but also can distinguish these signals from noise typical in current sequencing methods. Increases in sensitivity and specificity from blood-based samples of cell-free nucleic acids can be achieved using various methods. One method includes high efficiency tagging of nucleic acid molecules in the sample, tagging at least any of 50%, 75% or 90% of the polynucleotides in a sample. This increases the likelihood that a low-abundance target molecule in a sample will be tagged and subsequently sequenced, and significantly increases sensitivity of detection of target molecules.

Another method involves molecular tracking, which identifies sequence reads that have been redundantly generated from an original parent molecule, and assigns the most likely identity of a base at each locus or position in the parent molecule. This significantly increases specificity of detection by reducing noise generated by amplification and sequencing errors, which reduces frequency of false positives.

Methods of the present disclosure can be used to detect genetic variation in non-uniquely tagged initial starting genetic material (rare nucleic acids) at a concentration that is less than 5%, 1%, 0.5%, 0.1%, 0.05%, or 0.01%, at a specificity of at least 99%, 99.9%, 99.99%, 99.999%, 99.9999%, or 99.99999%. Sequence reads of tagged polynucleotides can be subsequently tracked to generate consensus sequences for polynucleotides with an error rate of no more than 2%, 1%, 0.1%, or 0.01%.

### V. Gene Fusion Events and Breakpoint Regions

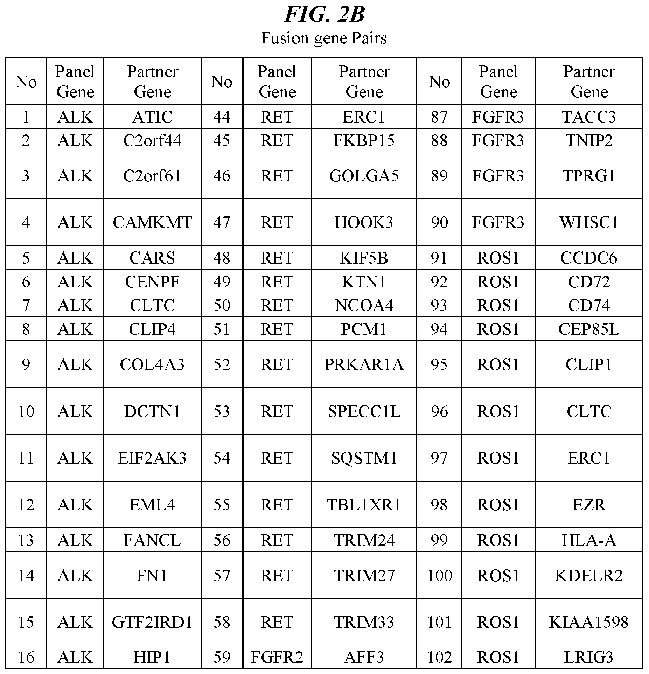

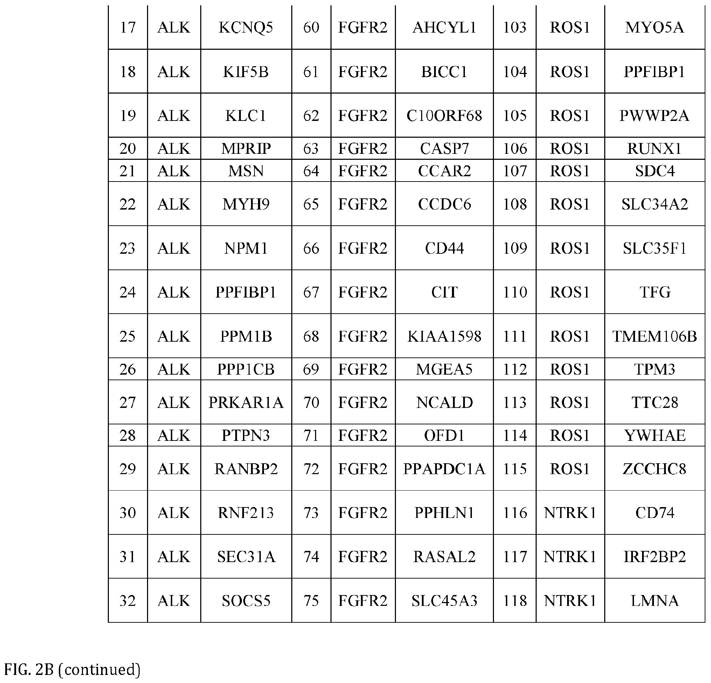

Gene fusion events are chromosomal rearrangements (inversion, deletion, and translocation) that bring together formerly separate portions of at least two different genes in a genome, resulting in a fusion gene. Fusion genes can be associated with and/or cause the formation of a neoplasm. A fusion gene can be a cancer fusion gene. A cancer fusion gene can be a fusion gene resulting from a somatic mutation that is present in a cancer. Non-limiting examples of pairs of genes that may form cancer fusion genes are found in **FIGs. 2A** and **2B****.** Non-limiting examples of genes involved in fusion genes are listed in **FIG. 3****.**

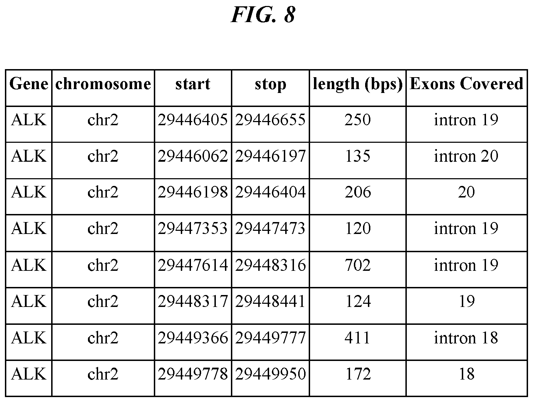

**FIG. 8** shows non-limiting examples of genomic regions of the ALK gene that may be targeted for deeper coverage. The genomic regions in **FIG. 8** may correspond to different variants of the ALK gene. Such deep coverage may be quantified by the number of unique molecules obtained after sequencing and collapsing with molecular barcodes, e.g., about 2-3 thousand molecules for typical variants versus about 4 thousand molecules for the genomic regions of **FIG. 8****.** A range of a few thousand unique molecules may corresponds to greater than 1000x, 2000x, 3000x, 4000x, 5000x, or 10,000x sequencing depth.

Typically, a fusion gene can result in an aberrant juxtaposition of two genes that can encode a fusion protein (BCR-ABLl), or the regulatory elements of one gene may drive the aberrant expression of an oncogene (TMPRSS2-ERG). Despite the recurrent nature of cancer fusion genes, the exact location of the breakpoint for each fusion gene can vary. A breakpoint region refers to a region of a gene that may be involved in gene fusions at which a breakpoint can occur. In some cases, the breakpoint region is at most within 500 nucleotides of a breakpoint. In some cases, the breakpoint region is within at most 200 nucleotides of a breakpoint, within at most 500 nucleotides of a breakpoint, within at most 750 nucleotides of a breakpoint, within at most 1 kilobase (kb) of a breakpoint, within at most 5 kb of a breakpoint, within at most 10 kb of a breakpoint, within at most 20 kb of a breakpoint, within at most 30 kb of a breakpoint, within at most 40 kb of a breakpoint, within at most 50 kb of a breakpoint, or within at most 100 kb of a breakpoint.

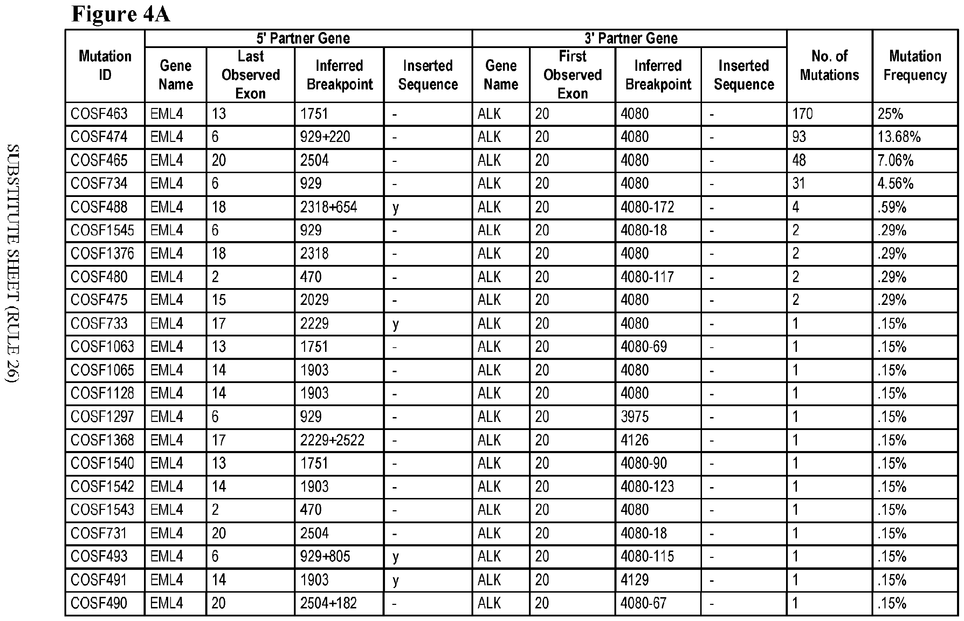

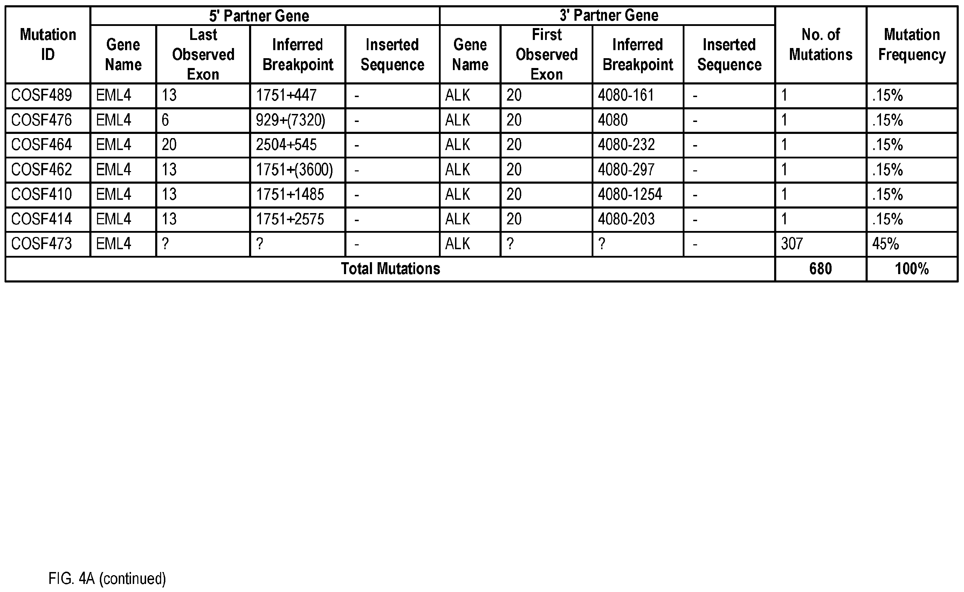

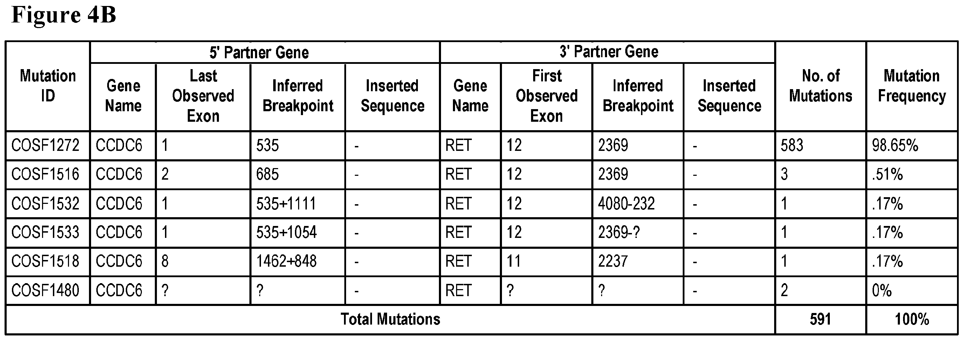

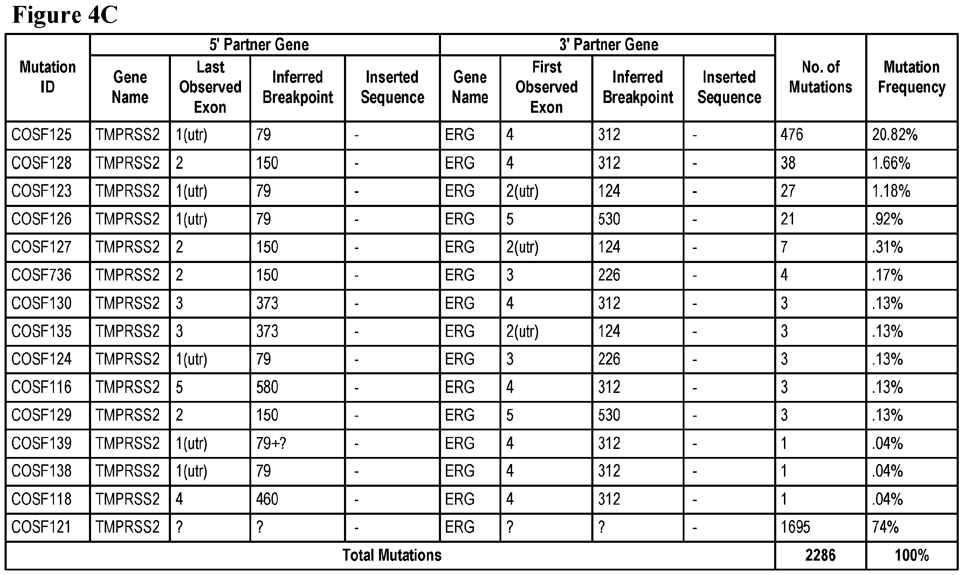

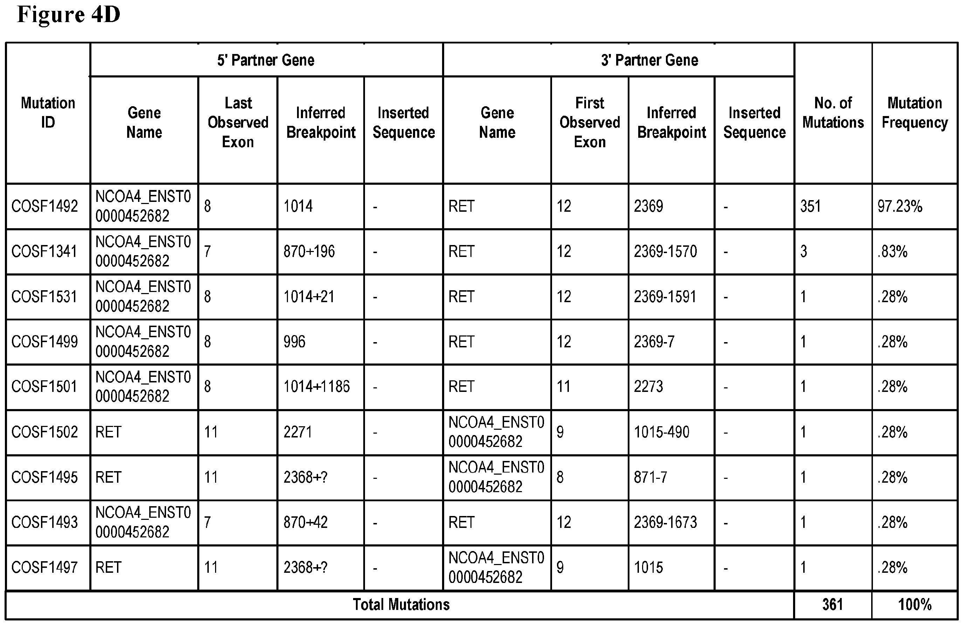

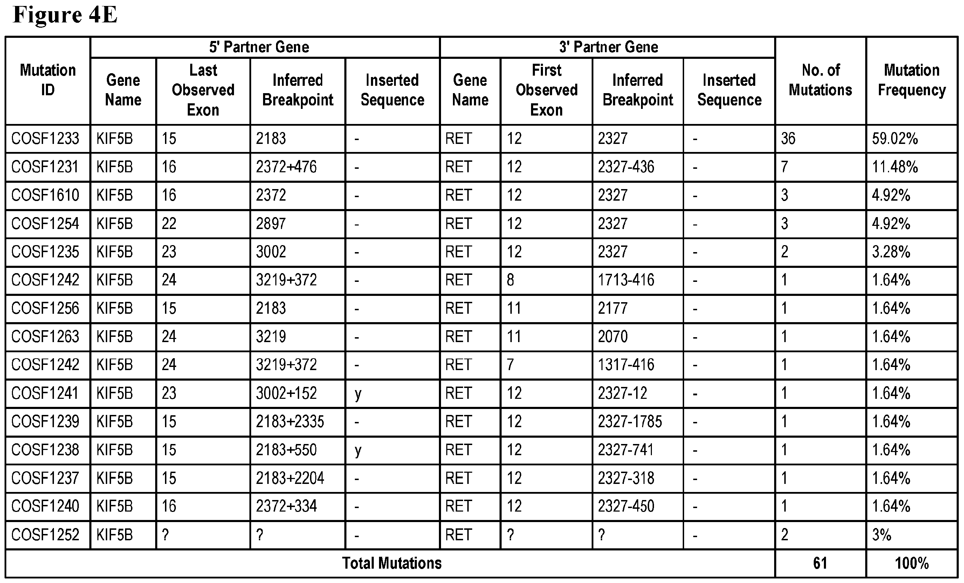

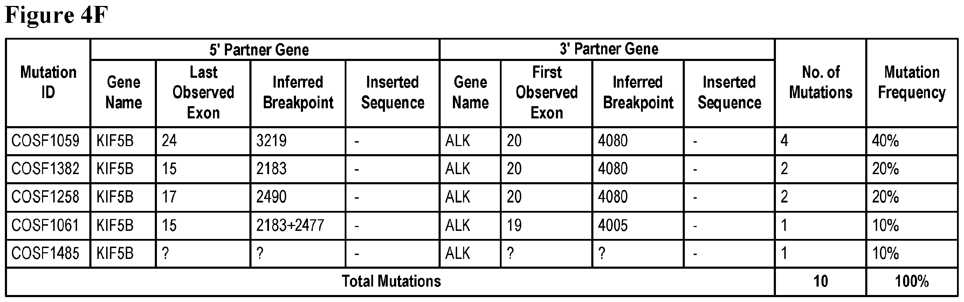

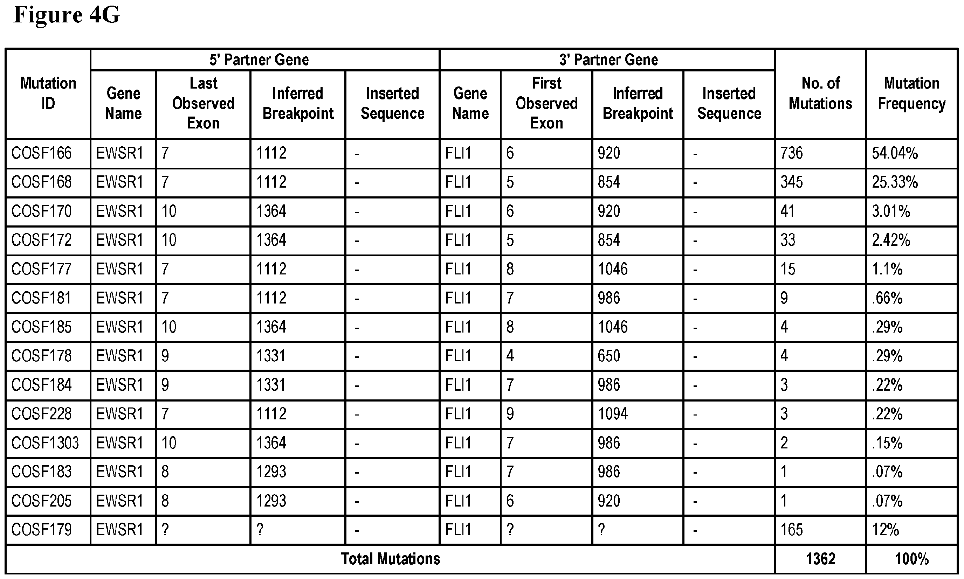

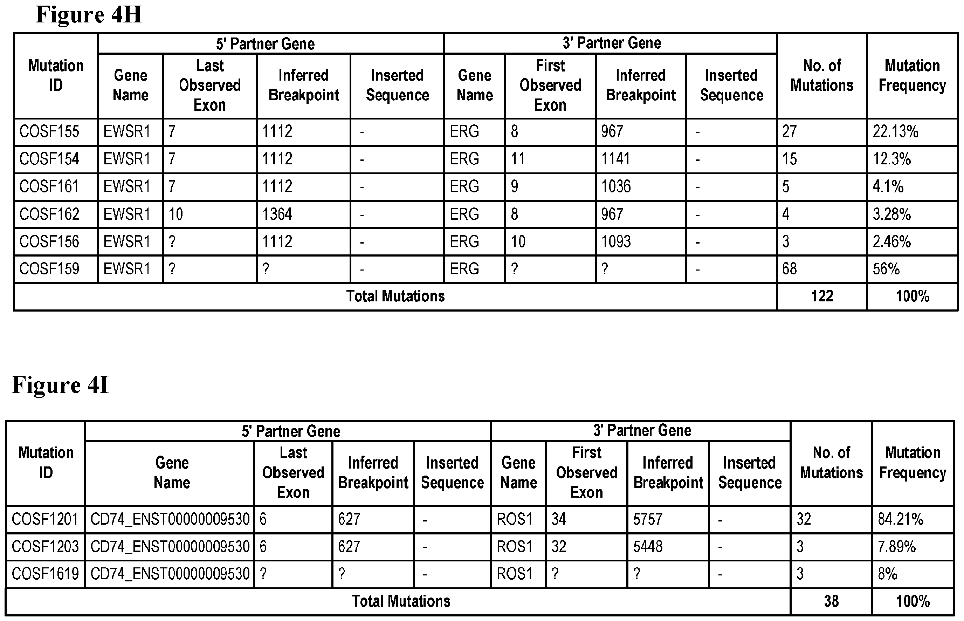

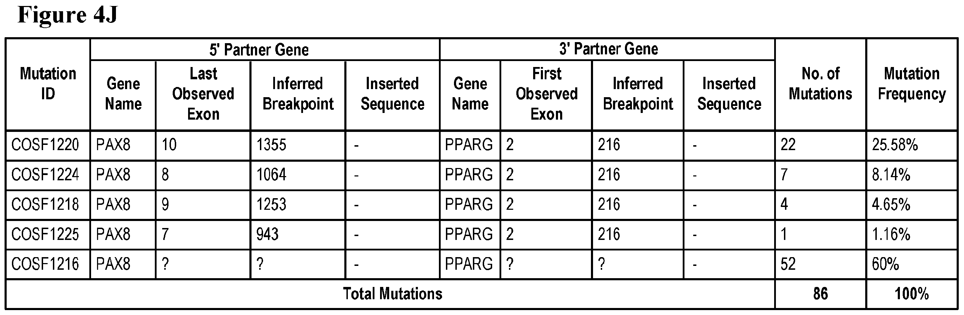

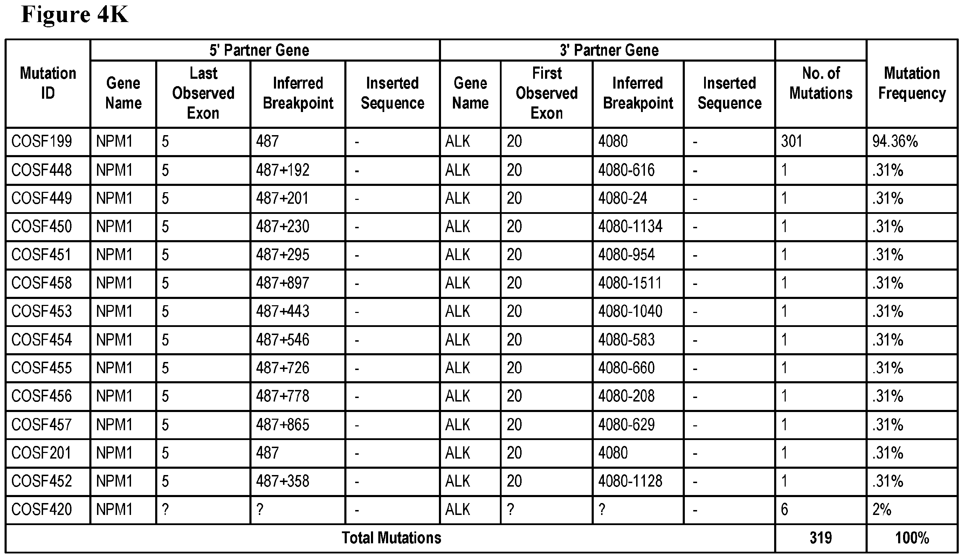

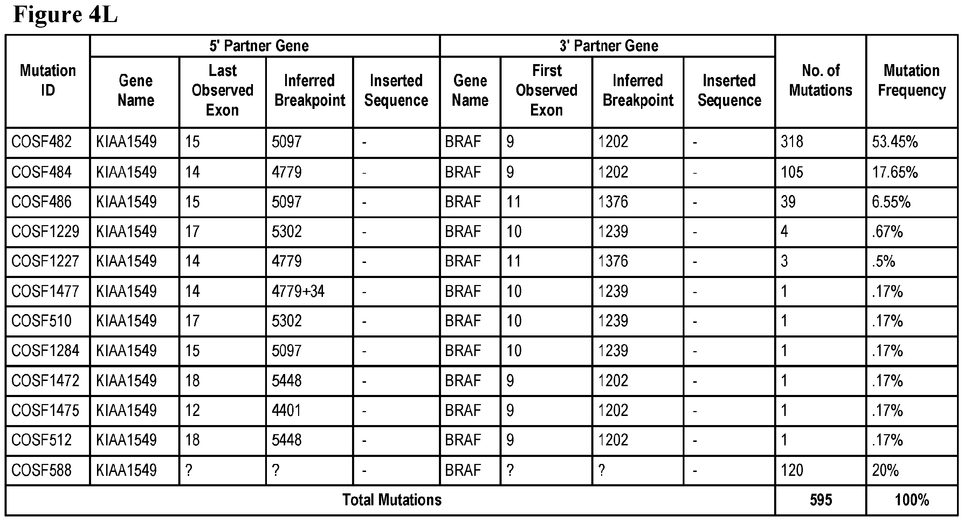

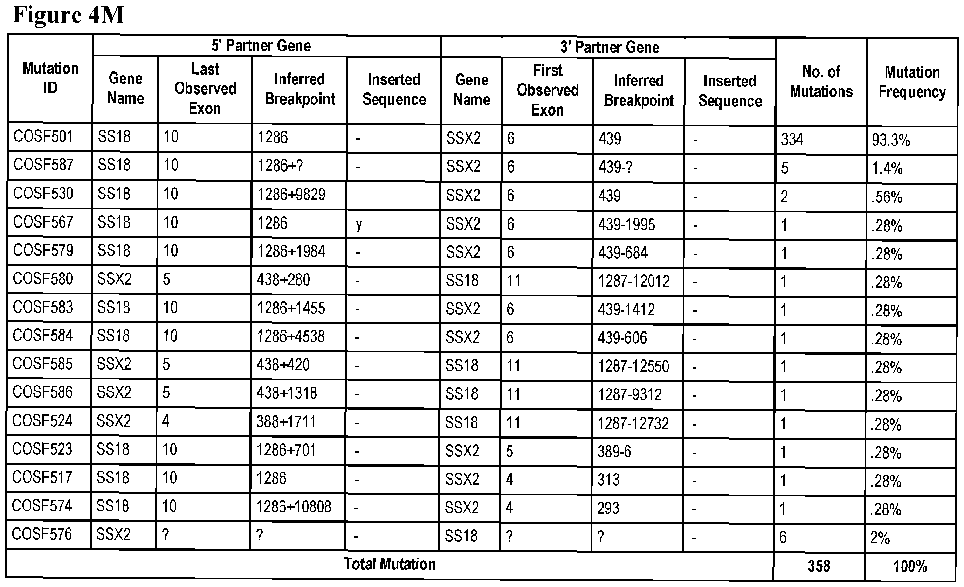

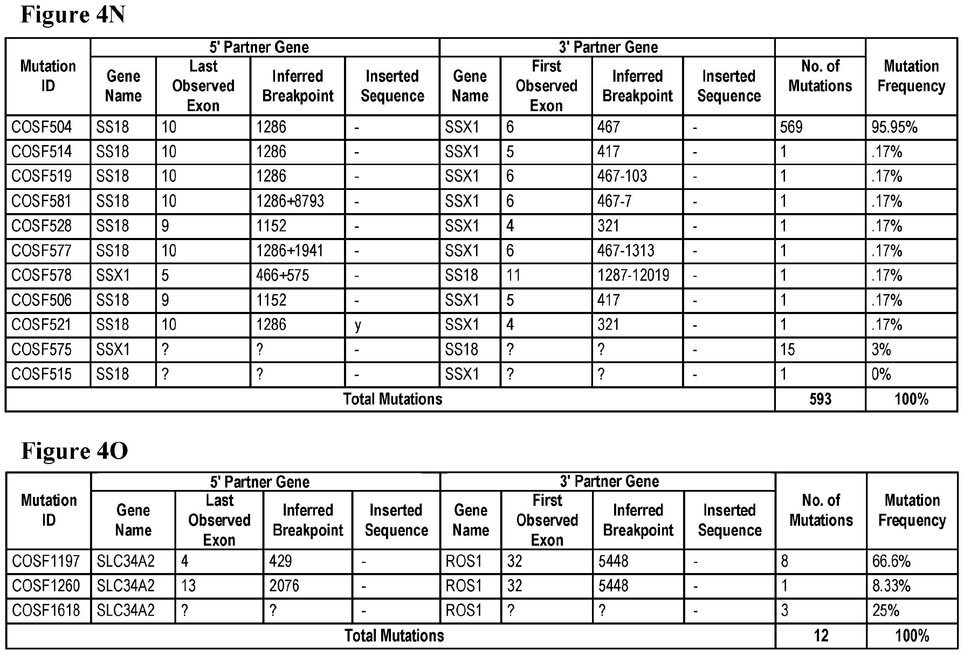

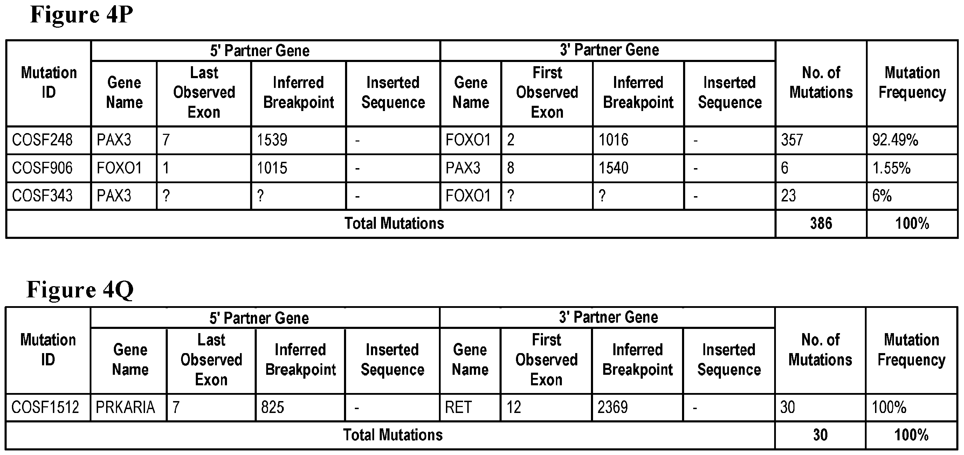

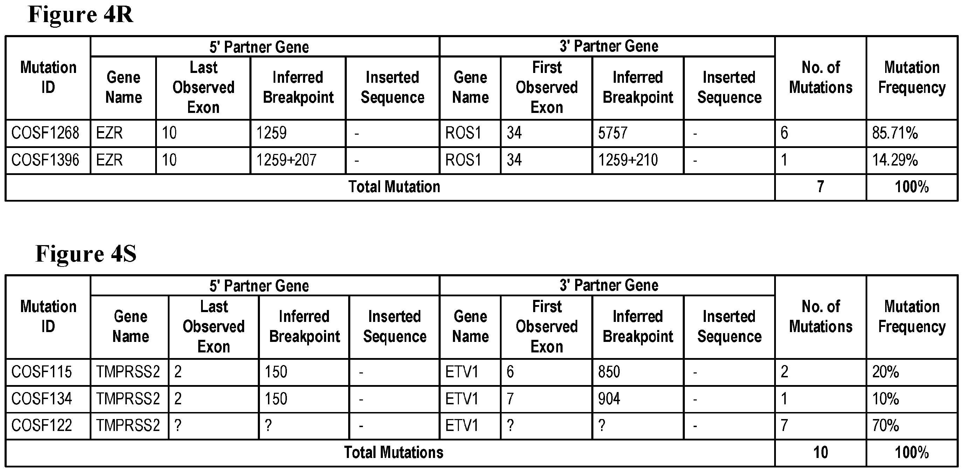

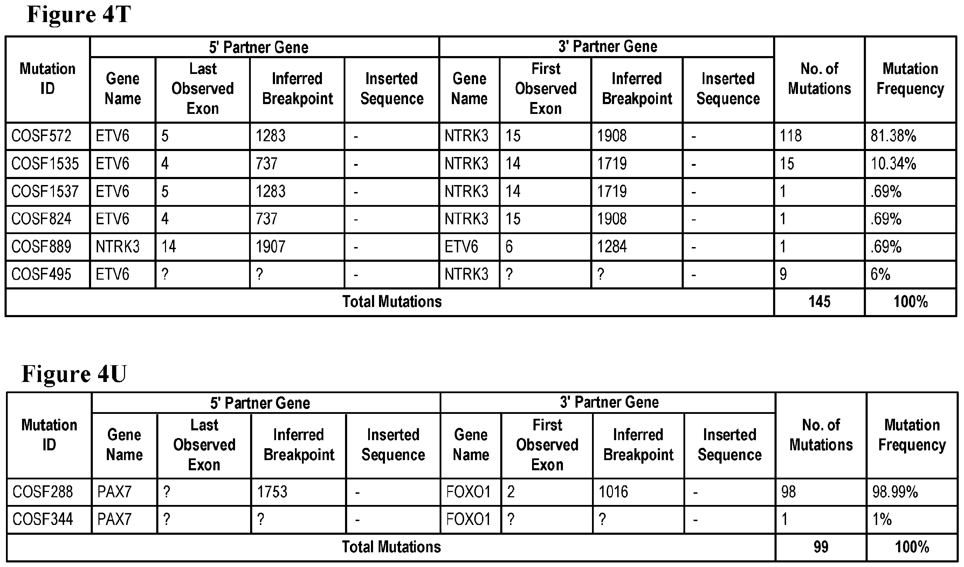

Exemplary, non-limiting breakpoints for given pairs of genes are provided in **FIG. 4A-4U** from the Catalogue of Somatic Mutations in Cancer (COSMIC; see Forbes et al., Nucleic Acids Research (2014) 43:D805-D811). For each gene pair, a specific mutation ID is provided in the first column that indicates a particular class of detected or inferred fusion construct from the literature. For example, **FIG. 4A** provides 29 classes of detected or inferred fusion constructs from the literature. For each mutation, the 5' and 3' fusion partner (5' and 3' are relative to the directionality of each gene's transcript) each provide the gene name, the last observed exon, the inferred breakpoint relative to the transcript, whether there and whether there is inserted sequence. For each mutation ID, a number of unique samples observed with the mutation and the percentage of gene fusions involving the two genes that have that particular mutation are also provided.

For example, the first row of **FIG. 4A** indicates that Mutation COSF463 is an EML4-ALK fusion, wherein the EML4 gene has fused upstream of the ALK gene. In this example, the last observed EML4 exon is exon 13, and the inferred breakpoint is at the genomic position corresponding to position 1751 of the EML4 gene transcript. The EML4 gene has fused such that the first ALK exon after the fusion junction is exon 20, and the inferred breakpoint position is the genomic position corresponding to position 4080 of the ALK gene transcript. There is no additional inserted sequence in either the 5' partner or 3' partner gene. The COSF463 fusion gene has been detected in 170 unique samples, or 25% of all EML4-ALK fusion genes included in the COSMIC database. In some instances, such COSF488 **(****FIG. 4A****,** row 5), the inferred breakpoint includes a '+' followed by a number, denoting a genomic position that number of bases downstream (in an intron or UTR) of the transcript position indicated by the first number. If the number is in parentheses the position is approximate. In some instances, such COSF488 **(****FIG. 4A****,** row 5), the inferred breakpoint includes a '-' followed by a number, denoting a genomic position that number of bases upstream (in an intron or UTR) of the transcript position indicated by the first number. If the number is in parentheses the position is approximate. A '?' indicates that the precise breakpoint is unknown. For example, in COSF488, the breakpoint is 654 bases downstream of the genomic position corresponding to position 2318 of the EML4 gene transcript, which has fused to a position 172 bases upstream of the genomic position corresponding to position 4080 of the ALK gene transcript.

### VI. High Affinity Polynucleotides

In some cases, the high affinity polynucleotide can be at least about 450 nucleotides in length, at least about 425 nucleotides in length, at least about 400 nucleotides in length, at least about 375 nucleotides in length, at least about 350 nucleotides in length, at least about 325 nucleotides in length, at least about 300 nucleotides in length, at least about 275 nucleotides in length, at least about 250 nucleotides in length, at least about 225 nucleotides in length, at least about 200 nucleotides in length, at least about 180 nucleotides in length, at least about 160 nucleotides in length, at least about 140 nucleotides in length, at least about 120 nucleotides in length, at least about 100 nucleotides in length, at least about 80 nucleotides in length, at least about 60 nucleotides in length, at least about 40 nucleotides in length, or at least about 20 nucleotides in length.

Furthermore, in some cases, the high affinity polynucleotide can be at most about 500 nucleotides in length, at most about 450 nucleotides in length, at most about 425 nucleotides in length, at most about 400 nucleotides in length, at most about 375 nucleotides in length, at most about 350 nucleotides in length, at most about 325 nucleotides in length, at most about 300 nucleotides in length, at most about 275 nucleotides in length, at most about 250 nucleotides in length, at most about 225 nucleotides in length, at most about 200 nucleotides in length, at most about 180 nucleotides in length, at most about 160 nucleotides in length, at most about 140 nucleotides in length, at most about 120 nucleotides in length, at most about 100 nucleotides in length, at most about 80 nucleotides in length, at most about 60 nucleotides in length, at most about 40 nucleotides in length, or at most about 20 nucleotides in length.

In particular, in some cases high affinity polynucleotides can be between about 20 and about 200 nucleotides in length. Furthermore, in some cases high affinity polynucleotides can be between about 80 and about 160 nucleotides in length.

In certain embodiments, high affinity polynucleotides of this invention have a sequence of at least 10, least 25, least 50, least 100 or at least 150 nucleotides perfectly complementary or substantially complementary to a target sequence of a fusion gene.

High affinity polynucleotides can contain one or more LNA nucleotides. In some cases, 100% of the nucleotides within the high affinity polynucleotide are LNA nucleotides. In some cases, at least 90%, at least 70%, at least 50%, at least 20%, at least 10%, at least 5%, or at least 1% of the nucleotides within the high affinity polynucleotides are LNA nucleotides. In some cases, at most 90%, at most 70%, at most 50%, at most 20%, at most 10%, at most 5%, or at most 1% of the nucleotides within the high affinity polynucleotide are LNA nucleotides.

If a high affinity polynucleotide contains more than one LNA nucleotide, in some cases the LNA nucleotides can be spaced no more than 30 nucleotides apart, no more than 20 nucleotides apart, no more than 15 nucleotides apart, no more than 10 nucleotides apart, or no more than 5 nucleotides apart. In other cases where the high affinity polynucleotide contains more than one LNA nucleotide, the LNA nucleotides can be spaced at least 30 nucleotides apart, at least 20 nucleotides apart, at least 15 nucleotides apart, at least 10 nucleotides apart, or at least 5 nucleotides apart.

For each LNA nucleotide inserted in place of a natural nucleotide in a high affinity polynucleotide, the melting temperature of the duplex of the high affinity polynucleotide and its complementary sequence comprising only natural nucleotides can increase at least 1 °C, at least 2 °C, at least 3 °C, 4 at least °C, at least 5 °C, at least 6 °C, at least 7 °C, at least 8 °C, at least 9 °C, or at least 10 °C under stringent conditions. In particular, for each LNA nucleotide inserted in place of a natural nucleotide, the melting temperature can increase by between about 2 °C and about 8 °C.

In some cases, the melting temperature of a high affinity polynucleotide (comprising one or more LNA nucleotides) can be at least 0.5% higher, at least 1% higher, at least 2% higher, at least 3% higher, at least 4% higher, at least 5% higher, at least 10% higher, at least 15% higher, at least 20% higher, at least 25% higher, at least 30% higher, at least 35% higher, at least 40% higher, at least 45% higher, at least 50% higher, at least 55% higher, at least 60% higher, at least 65% higher, at least 70% higher, at least 75% higher, at least 80% higher, at least 85% higher, at least 90% higher, at least 95% higher, or at least 100% higher than the melting temperature of a polynucleotide comprising only natural nucleotides with the same sequence as the high affinity polynucleotide.

In one configuration, bound probes may be affinity purified using a combination of binding partners. In one example, probes may contain a binding partner such as biotin. The binding partner may then be used as bait for an additional binding partner, such as streptavidin, in an affinity purification step. In some cases, bound probes may be affinity purified from unbound probes. In other cases, sample polynucleotide strands, comprising a binding partner and bound probes may be affinity purified from unbound probes.

Generally, any chemical approach for capture of the bound probes may be suitable. In some cases, capture may be achieved through methods comprising biotin and streptavidin, or streptavidin derivatives. For example, one embodiment of the disclosure provides for capture of sequencing library fragments of fusion genes, wherein probes to the genes involved in the fusion gene, probes to the breakpoint region, and/or probes to the breakpoint are annealed to melted strands of the sequencing library and affinity purified away from other sequencing library fragments.

Magnetically attractable particles, such as beads, may be used for isolation. Any suitable bead isolation technique can be used with methods of the present disclosure. In some cases, Beads can be useful for isolation in that molecules of interest can be attached to the beads, and the beads can be washed to remove solution components not attached to the beads, allowing for enrichment, purification and/or isolation. The beads can be separated from other components in the solution based on properties such as size, density, or dielectric, ionic, and magnetic properties. In preferred embodiments, the particles are magnetically attractable. Magnetically attractable particles can be introduced, mixed, removed, and released into solution using magnetic fields. Processes utilizing magnetically attractable particles can also be automated. Magnetically attractable particles are supplied by a number of vendors including NEB, Dynal, Micromod, Turbobeads, and Spherotech. The particles can be functionalized using functionalization chemistry to provide a surface having the binding groups required for binding to polynucleotides.

In some cases, the probe and/or high affinity polynucleotide are configured to hybridize to a cancer fusion gene. For example, the probe and/or high affinity polynucleotide can be complementary to a portion of either gene that the fusion gene is derived from. In some cases, the cancer fusion gene can be one or more genes selected from the lists present in **FIGs. 2A-2B****.**

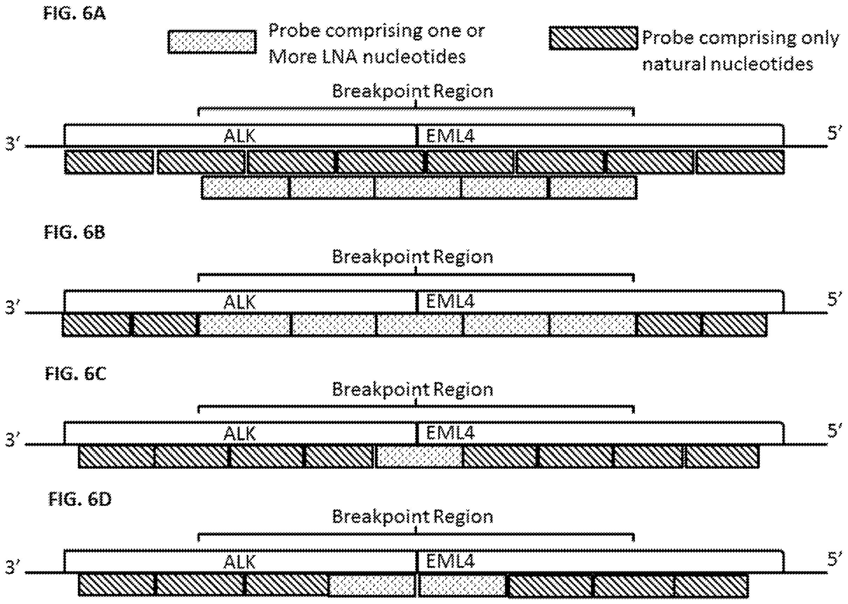

In some cases, the probe and/or high affinity polynucleotide can be configured to hybridize to a breakpoint region. For example, in some cases the probe and/or high affinity polynucleotide can be complementary to a portion of a breakpoint region (the probe and/or high affinity polynucleotide can be complementary to a sequence within 500 nucleotides of a breakpoint). Furthermore, in some cases, the probe and/or high affinity polynucleotide can be configured to hybridize across a breakpoint in a fusion gene (see **FIG. 6C****).** For example, the probe and/or polynucleotide can be complementary to a portion of the sequence on each side of a breakpoint (see **FIG. 6D****).**

### VII. Sets of Probes and/or Polynucleotides

In some cases, sets of probes and/or polynucleotides are provided. In some cases, all of the probes and/or polynucleotides in the set comprise LNA nucleotides. In some cases, a subset of the probes and/or polynucleotides in the set comprises only natural nucleotides, referred to hereafter as a "standard affinity subset", and a second subset comprising one or more LNA nucleotides, referred to hereafter as a "high affinity subset."

In one embodiment, the probe set includes one or more probes directed to a nucleotide sequence in a breakpoint region of a fusion gene.

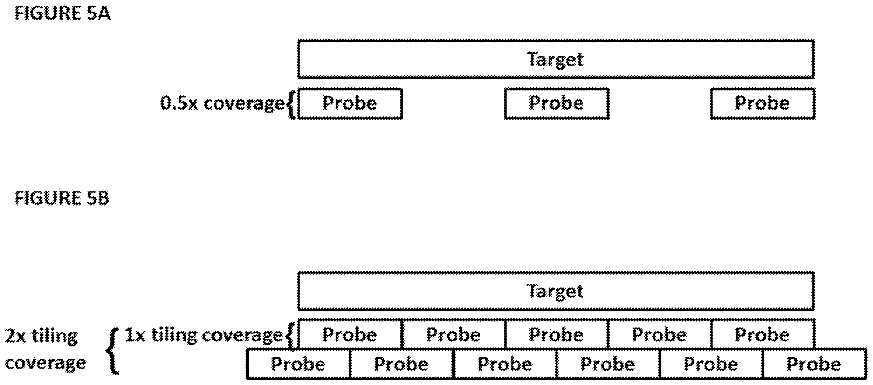

Probes and/or polynucleotides can be provided at a variety of coverage depths. For example, in some cases coverage depth can be at least 0.5x, wherein a set of probes or polynucleotides targets on average half of the bases in a region (see **FIG. 5A****).**

In some cases, coverage depth can be at least 1x, wherein probes and/or polynucleotides are designed such that each base in a region is on average targeted by only one probes and/or polynucleotide sequence. In some cases, coverage depth can be at least 2x, wherein probes and/or polynucleotides are designed such that each base in a region is on average targeted by two probes and/or polynucleotide sequences. In some cases, coverage depth by a set of probes or polynucleotides can be at least 3x, at least 4x, or at least 5x. In some cases, probes and/or polynucleotides can be tiling, wherein a set of probes and/or polynucleotides are designed such that a contiguous target region is covered by the probes and/or polynucleotide sequences (see **FIG. 5B****).**

In some cases, it may be preferable to use a standard affinity subset of probes and/or polynucleotides to enrich for some nucleic acid fragments of interest, and to use a high affinity subset of probes and/or polynucleotides to enrich for other nucleic acid fragments in the same sample. For example, in some cases a standard affinity subset of probes and/or polynucleotides can target exomes, oncogenes, or tumor suppressor genes, and a high affinity subset of probes and/or polynucleotides can target fusion genes, such as cancer fusion genes (e.g. the genes listed in **FIG. 3****).** In another example, in some cases, a standard affinity subset targets with a first coverage depth a contiguous or non-contiguous portion of one or more genes involved in a gene fusion, including the breakpoint regions, and a high affinity subset targets with a second coverage depth the breakpoint region(s) (see **FIG. 6A****).** In some cases, a standard affinity subset targets with a first coverage depth a contiguous or non-contiguous portion of each of the genes, excluding the breakpoint regions, and a high affinity subset targets with a second coverage depth the breakpoint region(s) (see **FIG. 6B****).** In some cases, a standard affinity subset targets with a first coverage depth a contiguous or non-contiguous portion of each of the genes, and a high affinity subset targets with a second coverage depth the breakpoints (see **FIG. 6C****).** In some cases, a standard affinity subset targets with a first coverage depth a contiguous or non-contiguous portion of each of the genes, and a high affinity subset targets with a second coverage depth the sequence on either side of a breakpoint, but not the breakpoint itself (see **FIG. 6D****).**

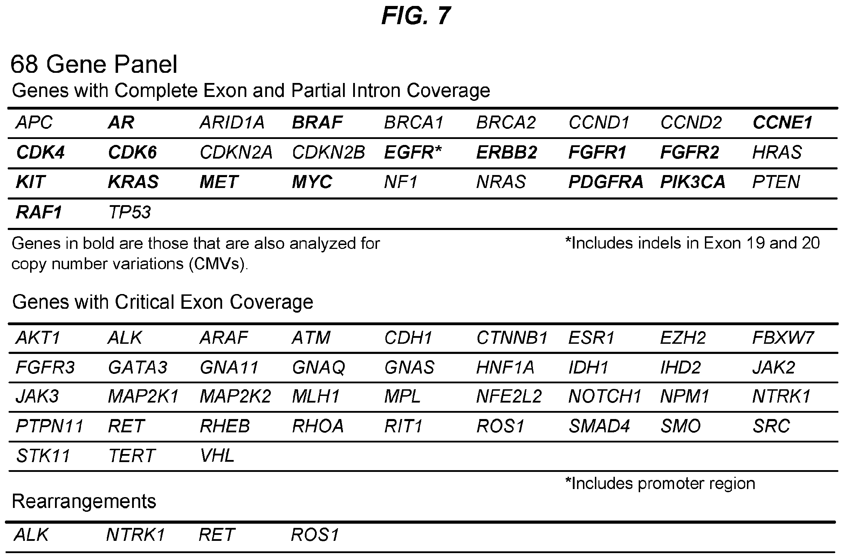

In some cases, a set of probes and/or polynucleotides is configured to target more than one gene in order to enrich for a panel of genes that may be involved in gene fusions (see, e.g., FIG. 7). Furthermore, in some cases, a set of probes and/or polynucleotides is configured to target more than one gene and their breakpoints or breakpoint regions.

In some cases, sets of probes and/or polynucleotides are configured to target a specific fusion gene. For example, the probes and/or polynucleotides can be designed to target one or both genes involved in the gene fusion. In some cases, a set of probes and/or polynucleotides comprises probes and/or polynucleotides that target a single gene and/or its breakpoints or breakpoint regions.

In some cases, the standard affinity probes and/or polynucleotides are mixed with the high affinity probes and/or polynucleotides. In some cases, the standard affinity probes and/or polynucleotides and the high affinity probes and/or polynucleotides are separate and employed sequentially. Furthermore, in some cases the sample is first contacted with the standard affinity probes, and then the uncaptured nucleic acid fragments are contacted with the high affinity probes.

In some cases, high affinity probe sets can include standard affinity polynucleotides doped with high affinity polynucleotides. In such a probe set, a target sequence can be targeted for hybridization by both standard and high affinity polynucleotides. In such a doped set, the high affinity polynucleotides can target only sequences at a breakpoint region.

### VIII. Kits

The present disclosure provides kits for enriching samples for breakpoint fragments. The kits can comprise any of the probes and/or polynucleotides disclosed herein. In some cases, the kit can comprise a plurality of probe sets, wherein each probe set hybridizes to a different gene and at least one of the probe sets is configured to hybridize to a fusion gene and comprises one or more high affinity polynucleotides and/or probes.

### IX. Methods of Use

The present disclosure provides methods for enriching for breakpoint fragments using any of the probes and/or polynucleotides disclosed herein. Such methods can comprise contacting a probe set that hybridizes to a fusion gene, wherein one or more probes and/or polynucleotides is a high affinity polynucleotide and/or probe, with a mixture of polynucleotides to produce probe-captured polynucleotides. The probe-captured polynucleotides can then be isolated to produce a sample enriched for polynucleotides comprising breakpoint fragments of the fusion gene. In some cases, the polynucleotides are cell-free DNA. In some cases, the polynucleotides are fragmented genomic DNA. In some cases, the probe-captured polynucleotides are eluted to isolate the captured polynucleotides form the probes. In some cases, the eluted polynucleotides are directly sequenced or used to produce sequencing libraries.

Methods of detecting fusion genes are provided. In a method, at least one probe set comprising at least one high affinity polynucleotide is provided that is directed to a gene involved in a gene fusion. The probe set can include both standard affinity and high affinity polynucleotide probes. In some embodiments, the probe set comprises a plurality of probe subsets, each subset directed to sequences of a different gene of interest, one or more of which genes are involved in a gene fusion in cancer and, in some examples, at least of which genes is not involved in a gene fusion.

The probe set may be mixed with a sample comprising DNA, such as cfDNA, under stringent hybridization conditions, and the DNA may be allowed to hybridize to the probes. Because the probe set includes high affinity polynucleotide probes, the probability of capturing DNA fragments including a fusion gene break point is increased. Captured DNA may be isolated from the probe and sequenced. Sequences may be analyzed to detect DNA fragments having sequences that span a breakpoint, such as DNA fragments that include sequences from two different genes normally not fused. The presence of fusion genes may be correlated with a disease, such as cancer. Accordingly, this method is useful in the diagnosis of the disease, such as cancer.

### Computer control systems

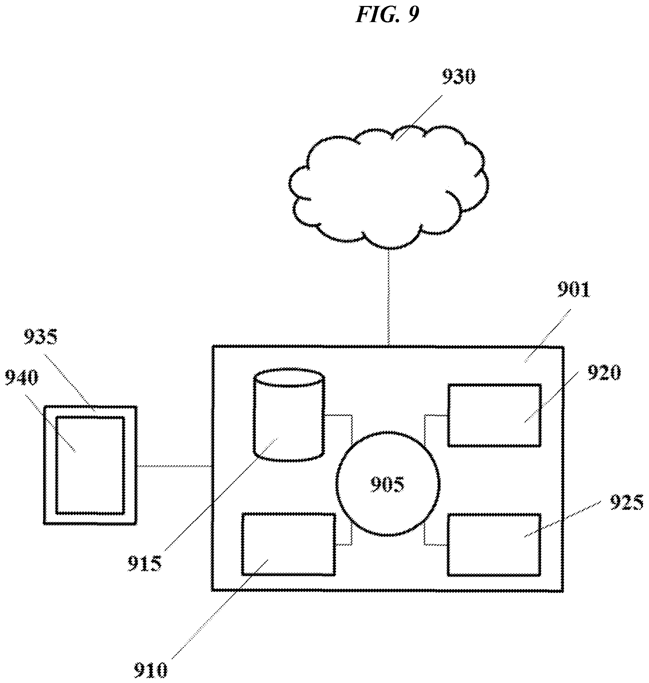

The present disclosure provides computer control systems that are programmed to implement methods of the disclosure. **FIG. 9** shows a computer system 901 that is programmed or otherwise configured to detect fusion genes and diagnose and/or provided a therapeutic intervention for a disease, such as cancer.

The computer system 901 includes a central processing unit (CPU, also "processor" and "computer processor" herein) 905, which can be a single core or multi core processor, or a plurality of processors for parallel processing. The computer system 901 also includes memory or memory location 910 (e.g., random-access memory, read-only memory, flash memory), electronic storage unit 915 (e.g., hard disk), communication interface 920 (e.g., network adapter) for communicating with one or more other systems, and peripheral devices 925, such as cache, other memory, data storage and/or electronic display adapters. The memory 910, storage unit 915, interface 920 and peripheral devices 925 are in communication with the CPU 905 through a communication bus (solid lines), such as a motherboard. The storage unit 915 can be a data storage unit (or data repository) for storing data. The computer system 901 can be operatively coupled to a computer network ("network") 930 with the aid of the communication interface 920. The network 930 can be the Internet, an internet and/or extranet, or an intranet and/or extranet that is in communication with the Internet. The network 930 in some cases is a telecommunication and/or data network. The network 930 can include one or more computer servers, which can enable distributed computing, such as cloud computing. The network 930, in some cases with the aid of the computer system 901, can implement a peer-to-peer network, which may enable devices coupled to the computer system 901 to behave as a client or a server.

The CPU 905 can execute a sequence of machine-readable instructions, which can be embodied in a program or software. The instructions may be stored in a memory location, such as the memory 910. The instructions can be directed to the CPU 905, which can subsequently program or otherwise configure the CPU 905 to implement methods of the present disclosure. Examples of operations performed by the CPU 905 can include fetch, decode, execute, and writeback.

The CPU 905 can be part of a circuit, such as an integrated circuit. One or more other components of the system 901 can be included in the circuit. In some cases, the circuit is an application specific integrated circuit (ASIC).

The storage unit 915 can store files, such as drivers, libraries and saved programs. The storage unit 915 can store user data, e.g., user preferences and user programs. The computer system 901 in some cases can include one or more additional data storage units that are external to the computer system 901, such as located on a remote server that is in communication with the computer system 901 through an intranet or the Internet.

The computer system 901 can communicate with one or more remote computer systems through the network 930. For instance, the computer system 901 can communicate with a remote computer system of a user (e.g., healthcare provider). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 901 via the network 930.

Methods as described herein can be implemented by way of machine (e.g., computer processor) executable code stored on an electronic storage location of the computer system 901, such as, for example, on the memory 910 or electronic storage unit 915. The machine executable or machine readable code can be provided in the form of software. During use, the code can be executed by the processor 905. In some cases, the code can be retrieved from the storage unit 915 and stored on the memory 910 for ready access by the processor 905. In some situations, the electronic storage unit 915 can be precluded, and machine-executable instructions are stored on memory 910.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 901, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 901 can include or be in communication with an electronic display 935 that comprises a user interface (UI) 940 for providing, an output of a report, which may include a diagnosis of a subject or a therapeutic intervention for the subject. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 905. The algorithm can, for example, facilitate the enrichment, sequencing and/or detection of fusion genes.

### EXAMPLES

### Example 1: Enrichment and sequencing of cancer genes and cancer fusion genes

Circulating cell-free DNA is isolated from the plasma of a cancer patient using the QIAamp Circulating Nucleic Acid kit (Qiagen) per manufacturer's protocol, except that a double sided SPRI with AmpureXP beads (Beckman Coulter) is performed to removed fragments >500 bps and keep all lower molecular weight fragments. The resulting ∼160-bp cfDNA fragments (5 to 30 ng) are then end-repaired and ligated to adapters with molecular barcode tags and sequences required for downstream next-generation sequencing (HiSeq2500, Illumina). The ligated cfDNA is amplified over 10 cycles using primers complementary to the ligated adapter sequences.

To enrich for regions of interest, including fusion genes, the resulting cfDNA libraries are denatured at 95 °C and then hybridized at 65 °C first to oligos that block the added sequences and then to 120-nt biotinylated RNA oligos (Agilent Technologies) and also 120-nt biotinylated RNA/LNA or DNA/LNA oligos (Exiqon) in stringent hybridization buffer for 16 hours. The hybridization reactions are captured using streptavidin beads (Invitrogen), washed to remove non-targeted cfDNA fragments, and eluted using sodium hydroxide. The resulting enriched libraries are amplified for another 12 cycles and sequenced on a HiSeq2500 (Illumina).

### Example 2: Sequence Capture

Cell-free DNA is isolated from a cancer patient.

A probe set is provided that is configured to capture polynucleotides having sequences of 68 target genes, including four genes involved in gene rearrangements. The probe set comprises sub-sets each sub-set directed to one of the 68 genes in the panel. Each subset directed to a gene not involved in a gene rearrangement is standard affinity subset (includes only non-high affinity polynucleotides, polynucleotides with only natural nucleotides). Each subset directed to a gene involved in a gene rearrangement is a high affinity subset (includes at least one high affinity polynucleotide). The sets have 2X tiling across exons. In the high affinity subsets, high affinity polynucleotides are directed only to breakpoint regions of the gene. The high affinity subsets are doped with high affinity polynucleotides, so that both high affinity polynucleotides and standard affinity polynucleotides are directed to sequences in the breakpoint regions.

Cell-free DNA and the probe set are combined under stringent hybridization conditions and incubated overnight. The probe set with bound cfDNA is isolated from the mixture. Bound polynucleotides are separated from the probes and sequenced. Polynucleotides comprising sequences across a breakpoint are identified.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

The invention is further characterized by the following numbered non-limiting embodiments:
1. A method for providing a diagnostic or therapeutic intervention to a subject having or suspected of having cancer, comprising:
   (a) providing a biological sample comprising cell-free nucleic acid molecules from a subject;
   (b) contacting the cell-free nucleic acid molecules from the biological sample with a probe set under hybridization conditions sufficient to produce probe-captured polynucleotides, which probe set comprises a plurality of polynucleotide probes, wherein each of the plurality of polynucleotide probes has (i) sequence complementarity with a fusion gene and (ii) affinity for the fusion gene that is greater than a polynucleotide having sequence complementary with the fusion gene and containing only unmodified nucleotides;
   (c) isolating the probe-captured polynucleotides from the mixture, to produce a sample enriched with isolated polynucleotides comprising breakpoint fragments of the fusion gene;
   (d) sequencing the isolated polynucleotides to produce sequences;
   (e) detecting polynucleotides comprising breakpoints of fusion genes based on the sequences; and
   (f) providing the diagnostic or therapeutic intervention based on the detection of breakpoint fragments.
2. The method of embodiment 1, wherein each of the plurality of polynucleotide probes comprises one or more locked nucleic acid (LNA) nucleotides.
3. The method of embodiment 2, wherein each of the plurality of polynucleotide probes comprises a plurality LNA nucleotides, wherein at least two of the LNA nucleotides are spaced no more than 30 nucleotides apart.
4. The method of embodiment 3, wherein the at least two of the LNA nucleotides are spaced no more than 15 apart.
5. The method of embodiment 1, wherein at least 50% of the nucleotides of each of at least a subset of the plurality of polynucleotide probes are locked nucleic acid (LNA) nucleotides.
6. The method of embodiment 5, wherein at least 75% of the nucleotides of each of at least a subset of the plurality of polynucleotide probes are locked nucleic acid (LNA) nucleotides.
7. The method of embodiment 1, wherein each of the plurality of polynucleotide probes has a melting temperature that is at least about 1 °C higher than the polynucleotide having sequence complementary with the fusion gene and containing only unmodified nucleotides.
8. The method of embodiment 7, wherein the melting temperature is at least about 10 °C higher.
9. The method of embodiment 1, wherein each of the plurality of polynucleotide probes has a melting temperature that is at least about 2% higher than the polynucleotide having sequence complementary with the fusion gene and containing only unmodified nucleotides.
10. The method of embodiment 9, wherein the melting temperature is at least about 10% higher.
11. The method of embodiment 1, wherein the fusion gene is a cancer fusion gene.
12. The method of embodiment 1, wherein each of the plurality of polynucleotide probes has sequence complementarity with a gene of a fusion gene pair of FIGs. 2A-2B or a fusion gene between two or more genes selected from FIG. 3.
13. The method of embodiment 1, wherein each of the plurality of polynucleotide probes has sequence complementarity with a breakpoint region no more than 500 nucleotides away from a breakpoint of the fusion gene.
14. The method of embodiment 1, wherein each of the plurality of polynucleotide probes has sequence complementarity with a sequence across a breakpoint in the fusion gene.
15. The method of embodiment 1, wherein each of the plurality of polynucleotide probes has a length less than about 500 nucleotides.
16. The method of embodiment 1, wherein each of the plurality of polynucleotide probes has a length between about 20 and about 200 nucleotides.
17. The method of embodiment 1, wherein each of the plurality of polynucleotide probes has a length between about 80 and about 160 nucleotides.
18. The method of embodiment 1, wherein each of the breakpoint fragments has a length between about 140 nucleotides and 180 nucleotides.
19. The method of embodiment 1, wherein the plurality of polynucleotide probes is coupled to a solid support.
20. The method of embodiment 1, wherein the probe set comprises one or more natural polynucleotide probes.
21. The method of embodiment 1, wherein the plurality of polynucleotide probes comprises at least one polynucleotide probe that hybridizes to a breakpoint region of a nucleic acid sequence included in the fusion gene, and at least one natural polynucleotide probe that hybridizes to a non-breakpoint region of the nucleic acid sequence included in the fusion gene.
22. The method of embodiment 1, wherein each of the plurality of polynucleotide probes provides at least 50% coverage of a breakpoint region of a nucleic acid sequence included in the fusion gene.
23. The method of embodiment 1, wherein (d) comprises attaching, to the isolated polynucleotides, tags comprising barcodes having distinct barcode sequences to generate tagged parent polynucleotides.
24. The method of embodiment 23, further comprising amplifying the tagged parent polynucleotides to produce tagged progeny polynucleotides.
25. The method of embodiment 24, further comprising (i) sequencing the tagged progeny polynucleotides to produce sequence reads, wherein each sequence read comprises a barcode sequence and a sequence derived from a given one of the isolated polynucleotides, and (ii) grouping the sequence reads into families based at least on the barcode sequence.
26. The method of embodiment 25, further comprising comparing the sequence reads grouped within each family to determine consensus sequences for each family, wherein each of the consensus sequences corresponds to a unique polynucleotide among the tagged parent polynucleotides.
27. A method for capturing a breakpoint fragment of a fusion gene, comprising:
   (a) providing a biological sample containing or suspected of containing a cell-free nucleic acid molecule comprising the breakpoint fragment of the fusion gene; and
   (b) contacting the biological sample with a polynucleotide probe under conditions sufficient to:
      i. permit hybridization between the polynucleotide probe and the breakpoint fragment to provide a probe-captured polynucleotide in a mixture, which polynucleotide probe has sequence complementarity with the breakpoint fragment and has affinity for the fusion gene that is greater than a polynucleotide having sequence complementary with the fusion gene and containing only unmodified nucleotides; and
      ii. enrichment or isolation of the probe-captured polynucleotide from the mixture, wherein the polynucleotide probe has sequence complementarity with the breakpoint fragment.
28. The method of embodiment 27, wherein the polynucleotide probe comprises one or more locked nucleic acid (LNA) nucleotides.
29. The method of embodiment 28, wherein the polynucleotide probe comprises a plurality LNA nucleotides, wherein at least two of the LNA nucleotides are spaced no more than 30 nucleotides apart.
30. The method of embodiment 29, wherein the at least two of the LNA nucleotides are spaced no more than 15 nucleotides apart.
31. A probe set comprising a plurality of polynucleotide probes, wherein each of the polynucleotide probes has (i) sequence complementarity with a fusion gene as part of a cell-free nucleic acid molecule and (ii) affinity for the fusion gene that is greater than a polynucleotide having sequence complementary with the fusion gene and containing only unmodified nucleotides.
32. The probe set of embodiment 31, wherein each of the plurality of polynucleotide probes comprises one or more locked nucleic acid nucleotides.
33. The probe set of embodiment 31, wherein further comprising one or more natural polynucleotide probes.
34. The probe set of embodiment 31, wherein each of the plurality of polynucleotide probes comprises at least one polynucleotide probe that hybridizes to a breakpoint region of a nucleic acid sequence included in the fusion gene, and at least one natural polynucleotide probe that hybridizes to a non-breakpoint region of the nucleic acid sequence included in the fusion gene.
35. The probe set of embodiment 31, wherein each of the plurality of polynucleotide probes provides at least 50% coverage of a breakpoint region of a nucleic acid sequence included in the fusion gene.
36. The probe set of embodiment 31, wherein the plurality of polynucleotide probes hybridize to portions of one or both of the different genes in the fusion gene.
37. The probe set of embodiment 31, further comprising a solid support, wherein the plurality of polynucleotide probes is coupled to the solid support.
38. The probe set of embodiment 31, wherein each of the plurality of polynucleotide probes has a melting temperature that is at least about 1 °C higher than the polynucleotide having sequence complementary with the fusion gene and containing only unmodified nucleotides.
39. The probe set of embodiment 38, wherein the melting temperature is at least about 10 °C higher.
40. The probe set of embodiment 31, wherein each of the plurality of polynucleotide probes has a melting temperature that is at least about 2% higher than the polynucleotide having sequence complementary with the fusion gene and containing only unmodified nucleotides.
41. The probe set of embodiment 40, wherein the melting temperature is at least about 10% higher.
42. The probe set of embodiment 31, wherein the fusion gene is a cancer fusion gene.
43. The probe set of embodiment 31, wherein each of the plurality of polynucleotide probes has sequence complementarity with a gene of a fusion gene pair of FIGs. 2A-2B or a fusion gene between two or more genes selected from FIG. 3.
44. A high affinity polynucleotide, comprising a sequence that is configured to specifically hybridize to a nucleic acid sequence associated with a fusion gene in a cell-free nucleic acid molecule.
45. A high affinity polynucleotide configured to specifically hybridize to a fusion gene.
46. The high affinity polynucleotide of embodiment 45, wherein the high affinity polynucleotide comprises one or more locked nucleic acid nucleotides.
47. The high affinity polynucleotide of embodiment 45, wherein the high affinity polynucleotide has a melting temperature of at least 1 °C higher than a polynucleotide with the same sequence comprising only natural nucleotides.
48. The high affinity polynucleotide of embodiment 45, wherein the high affinity polynucleotide has a melting temperature that is at least 2% higher than a polynucleotide with the same sequence comprising only natural nucleotides.
49. The high affinity polynucleotide of embodiment 45, wherein the high affinity polynucleotide is configured to specifically hybridize to a cancer fusion gene.
50. The high affinity polynucleotide of embodiment 45, wherein the high affinity polynucleotide is configured to specifically hybridize to a gene of a fusion gene pair of FIGs. 2A-2B or a fusion gene between two or more genes selected from FIG. 3.
51. The high affinity polynucleotide of embodiment 45, wherein the high affinity polynucleotide is configured to hybridize within a breakpoint region no more than 500 nucleotides away from a breakpoint of the fusion gene.
52. The high affinity polynucleotide of embodiment 45, wherein the high affinity polynucleotide is configured to hybridize across a breakpoint in the fusion gene.
53. The high affinity polynucleotide of embodiment 45, wherein the high affinity polynucleotide has a length less than about 500 nucleotides.
54. The high affinity polynucleotide of embodiment 46, further comprising a plurality of locked nucleic acid (LNA) nucleotides, wherein at least two of the LNA nucleotides are spaced no more than 30 nucleotides apart.
55. The high affinity polynucleotide of embodiment 46, wherein at least 1% of the nucleotides in the polynucleotide are locked nucleic acid nucleotides.
56. The high affinity polynucleotide of embodiment 45, wherein the high affinity polynucleotide has a nucleotide sequence perfectly or substantially complementary to a nucleotide sequence of the fusion gene.
57. A high affinity polynucleotide probe comprising a high affinity polynucleotide configured to specifically hybridize to a fusion gene.
58. The probe of embodiment 57, wherein the high affinity polynucleotide comprises one or more locked nucleic acid nucleotides.
59. The probe of embodiment 57, further comprising a functionality selected from a detectable label, a binding moiety or a solid support.
60. The probe of embodiment 57, wherein the high affinity polynucleotide is configured to hybridize to a breakpoint fragment of a fusion gene.
61. The probe of embodiment 60, wherein the breakpoint fragment has a length between about 140 nucleotides and about 180 nucleotides.
62. The probe of embodiment 60, wherein the breakpoint fragment is cell-free DNA or genomic DNA.
63. The probe of embodiment 57, wherein the high affinity polynucleotide is bound to a solid support.
64. A probe set comprising a plurality of polynucleotide probes, each probe configured to specifically hybridize to a fusion gene, wherein the probe set comprises one or more high affinity polynucleotide probes.
65. The probe set of embodiment 64, wherein the high affinity polynucleotide comprises one or more locked nucleic acid nucleotides.
66. The probe set of embodiment 64, wherein the probe set comprises one or more natural polynucleotide probes.
67. The probe set of embodiment 64, further comprising at least one high affinity polynucleotide probe that specifically hybridizes to a breakpoint region of a gene involved in the fusion gene, and at least one natural polynucleotide probe that hybridizes to a non-breakpoint region of the gene involved in the fusion gene.
68. The probe set of embodiment 64, wherein the one or more high affinity polynucleotide probes in the probe set provide at least 50% (at least 0.5X to 5X) coverage of a breakpoint region of a gene involved in the fusion gene.
69. The probe set of embodiment 64, wherein the polynucleotide probes hybridize to portions of one or both of the different genes in the fusion gene.
70. The probe set of embodiment 64, wherein the probe set is configured as an oligonucleotide chip.
71. The probe set of embodiment 64, wherein a target sequence is targeted by both high affinity polynucleotide probes and standard affinity polynucleotide probes.
72. A kit comprising a plurality of probe sets, wherein each probe set specifically hybridizes to a different gene and at least one of the probe sets is a probe set of embodiment 64.
73. The kit of embodiment 72, wherein the high affinity polynucleotide comprises one or more locked nucleic acid nucleotides.
74. A method for capturing a breakpoint fragment of a fusion gene comprising contacting the breakpoint fragment with a high affinity polynucleotide probe under stringent hybridization conditions and allowing hybridization, wherein the polynucleotide probe is bound to a solid support and wherein the polynucleotide probe has a nucleotide sequence that is substantially or perfectly complementary to a nucleotide sequence of the breakpoint fragment.
75. The method of embodiment 74, wherein the high affinity polynucleotide comprises one or more locked nucleic acid nucleotides.
76. A method for enriching a sample for polynucleotides comprising a breakpoint of a fusion gene, comprising:
   (a) contacting a probe set of embodiment 64 with a mixture of polynucleotides under hybridization conditions to produce probe-captured polynucleotides; and
   (b) isolating the probe-captured polynucleotides from the mixture, to produce a sample enriched with polynucleotides comprising breakpoint fragments of the fusion gene.
77. The method of embodiment 76, wherein the high affinity polynucleotide comprises one or more locked nucleic acid nucleotides.
78. The method of embodiment 76, wherein the polynucleotides comprise cell-free DNA or fragmented genomic DNA
79. The method of embodiment 76, further comprising isolating captured polynucleotides from the probes.
80. The method of embodiment 76, further comprising sequencing the isolated polynucleotides.
81. A method of diagnosing cancer in a subject comprising:
   (a) providing a sample comprising polynucleotides from a subject;
   (b) contacting cell-free deoxyribonucleic acid from the sample with a probe set of embodiment 64 under hybridization conditions to produce probe-captured polynucleotides;
   (c) isolating the probe-captured polynucleotides from the mixture, to produce a sample enriched with polynucleotides comprising breakpoint fragments of the fusion gene;
   (d) sequencing the isolated polynucleotides to produce sequences;
   (e) detecting polynucleotides comprising breakpoints of fusion genes based on the sequences; and
   (f) diagnosing cancer based on the detection of breakpoint fragments.
82. The method of embodiment 81, wherein the high affinity polynucleotide comprises one or more locked nucleic acid nucleotides.

## Claims

1. A probe set comprising a plurality of polynucleotide probes, wherein each of the plurality of polynucleotide probes
(i) comprises one or more locked nucleic acid (LNA) nucleotides,
(ii) has sequence complementarity with a fusion gene as part of a cell-free nucleic acid molecule, and
(iii) has affinity for the fusion gene that is greater than a polynucleotide having sequence complementary with the fusion gene and containing only unmodified nucleotides.

2. The probe set of claim 1, wherein each of the plurality of polynucleotide probes comprises a plurality of LNA nucleotides, wherein at least two of the LNA nucleotides are spaced no more than 30 nucleotides apart.

3. The probe set of claim 2, wherein at least two of the LNA nucleotides are spaced no more than 15 nucleotides apart.

4. The probe set of any one of the preceding claims, further comprising one or more natural polynucleotide probes.

5. The probe set of claim 4, wherein each of the plurality of polynucleotide probes comprises at least one polynucleotide probe that hybridizes to a breakpoint region of a nucleic acid sequence included in the fusion gene, and at least one natural polynucleotide probe that hybridizes to a non-breakpoint region of the nucleic acid sequence included in the fusion gene.

6. The probe set of claim 1, wherein each of the plurality of polynucleotide probes provides at least 50% coverage of a breakpoint region of a nucleic acid sequence included in the fusion gene, wherein the breakpoint region is within at most 200 nucleotides of a breakpoint, within at most 500 nucleotides of a breakpoint, within at most 750 nucleotides of a breakpoint, within at most 1 kilobase (kb) of a breakpoint, within at most 5 kb of a breakpoint, within at most 10 kb of a breakpoint, within at most 20 kb of a breakpoint, within at most 30 kb of a breakpoint, within at most 40 kb of a breakpoint, within at most 50 kb of a breakpoint, or within at most 100 kb of a breakpoint.

7. The probe set of claim 3, wherein the breakpoint region is at most within 500 nucleotides of a breakpoint.

8. The probe set of claim 1, wherein the plurality of polynucleotide probes hybridize to portions of one or both of the different genes in the fusion gene.

9. The probe set of claim 1, further comprising a solid support, wherein the plurality of polynucleotide probes is coupled to the solid support.

10. The probe set of claim 1, wherein each of the plurality of polynucleotide probes has a melting temperature that is at least about 1 °C higher than the polynucleotide having sequence complementary with the fusion gene and containing only unmodified nucleotides.

11. The probe set of claim 8, wherein the melting temperature is at least about 10 °C higher.

12. The probe set of claim 1, wherein each of the plurality of polynucleotide probes has a melting temperature that is at least about 2 % higher than the polynucleotide having sequence complementary with the fusion gene and containing only unmodified nucleotides.

13. The probe set of claim 10, wherein the melting temperature is at least about 10% higher.

14. The probe set of claim 1, wherein the fusion gene is a cancer fusion gene.

15. The probe set of claim 1, wherein each of the plurality of polynucleotide probes has sequence complementarity with a gene of a fusion gene pair of FIGs. 2A-2B or a fusion gene between two or more genes selected from FIG. 3.
